(19) Europäisches Patentamt
European Patent Office
Office européen des brevets

(11) Numéro de publication : **0 518 757 A1**

(12) # DEMANDE DE BREVET EUROPEEN

(21) Numéro de dépôt : **92401601.7**

(22) Date de dépôt : **10.06.92**

(51) Int. Cl.$^5$ : **C12N 15/40,** A61K 39/12, C07K 15/00

(30) Priorité : **11.06.91 FR 9107077**

(43) Date de publication de la demande : **16.12.92 Bulletin 92/51**

(84) Etats contractants désignés : **AT BE CH DE DK ES FR GB GR IT LI LU NL PT SE**

(71) Demandeur : **SOCIETE EUROPEENNE DE BIOTECHNOLOGIE société anonyme de droit belge**
**Rue du Bois Saint-Jean, 14**
**B-4102 Seraing (Ougree) (BE)**

(71) Demandeur : **RHôNE MERIEUX S.A.**
**17, rue Bourgelat**
**F-69002 Lyon (FR)**

(72) Inventeur : **Lecomte, Corine, M. T. G.**
**15 rue de la Croix**
**B-4560 Ocquier (BE)**
Inventeur : **Renard, André, J. J.**
**Avenue des Cerfs 37**
**B-4031 Angleur (BE)**
Inventeur : **Chappuis, Gilles-Emile**
**3 rue Laurent Vibert**
**F-69006 Lyon (FR)**
Inventeur : **Pin, Jean-Jacques**
**Résidence Saint Bonnet, 94 Route Nationale**
**F-69720 Saint Bonnet de Mure (FR)**

(74) Mandataire : **Bernasconi, Jean et al**
**CABINET LEMOINE ET BERNASCONI 13, Boulevard des Batignolles**
**F-75008 Paris (FR)**

(54) **Peptides ou polypeptides du virus BVD et virus apparentés.**

(57) Les séquences nucléotidiques correspondant sensiblement à tout ou partie des fractions du génome du virus BVD codant respectivement pour les glycoprotéines gE2 et gE1, ou des combinaisons ou fragments de celles-ci, sont utilisées pour la réalisation de peptides et de virus recombinants incorporant ou exprimant ces peptides.

FIG.1

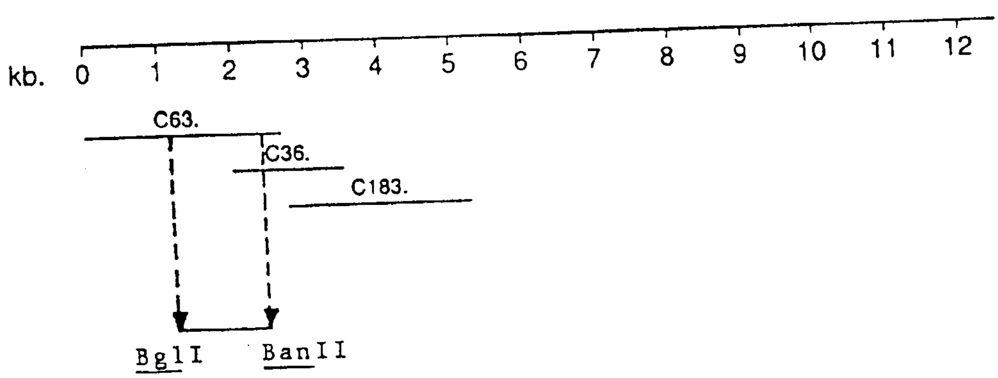


EP 0 518 757 A1

La présente invention a trait à de nouveaux peptides ou polypeptides immunogènes du virus de la diarrhée bovine et virus apparentés, en particulier virus de la maladie de Border, à des vaccins les incorporant ou les exprimant, ainsi qu'à des procédés et moyens de production y relatifs.

Le virus de la diarrhée bovine (BVD) est un virus enveloppé à ARN monocaténaire infectieux, qui est apparenté au virus de la peste porcine classique et à celui de la maladie de Border, les trois virus formant le genre Pestivirus qui appartient à la famille des Togaviridae. Le virus BVD est universellement répandu dans les populations bovines et se manifeste par un très large éventail de symptômes cliniques associés à des maladies congénitales, respiratoires ou entériques (diarrhée virale bovine, maladie des muqueuses).

Les isolats des virus BVD peuvent être classés en deux catégories ou biotypes distincts d'après leurs effets en culture de cellules : cytopathogène et non cytopathogène.

L'infection aiguë d'animaux séronégatifs est ordinairement bénigne ou subclinique. Par contre, une infection intra-utérine du foetus, dans les quatre premiers mois environ après le début de la gestation, par une souche non cytopathogène, peut non seulement produire des avortements, des mortinatalités ou la naissance de veaux faibles, mais aussi la naissance de veaux présentant une virémie persistante. Cette période correspond à une absence d'immunité chez le foetus. Lorsque le système immunitaire devient ensuite compétent, celui-ci reconnaît le virus comme sien et il s'établit une situation d'immunotolérance. Ces animaux ne pourront pas survivre à une infection ultérieure par une souche cytopathogène de virus BVD homologue.

Le maintien du virus non cytopathogène au sein de la population bovine est assuré par sa lente dissémination faisant suite à l'infection aiguë d'animaux séronégatifs et, surtout, par son excrétion continuelle par les animaux présentant une virémie persistante. Voir J. Brownlie et al., Ann. Rech. Vét. (1987) 18:157-166.

Des vaccins à virus atténués et à virus tués ont été développés dans le passé, sans résultats satisfaisants. Voir P. Saurat et al. "La Maladie des Muqueuses" (1972) : 229-251, l'Expansion scientifique française, Paris, et A.L. Fernelius et al., Am. J. Vet. Res. (1971) 32:1963-1979. Les vaccins à virus atténués peuvent être responsables de la mort de certains des animaux inoculés, notamment des animaux à virémie persistante. Les vaccins à virus tués quant à eux demandent de multiples inoculations et ne procurent qu'une immunité relativement faible et peu durable.

La recherche semble donc s'orienter vers d'autres types de vaccins, vaccins synthétiques et vaccins vivants recombinants. Pour ce faire, il faut avant tout rechercher des déterminants antigéniques.

Le génome de la souche virale Osloss de biotype cytopathogène a été cloné et entièrement séquencé par Renard et al. (demande de brevet EP-A-0.208.672 du 8 juillet 1985).

La demanderesse a trouvé que la phase de lecture ouverte (ORF), de la séquence génomique de BVD Osloss longue de 12408 nucléotides, a une capacité codante de 3951 acides aminés (aas).

Les protéines structurales sont codées par la partie 5' du génome et sont notamment une protéine non glycosylée de 270 acides aminés, appelée p20 suivant la nomenclature proposée par Collett et al. (Virology 165:200-208, 1988), et deux protéines glycosylées appelées gp62 et gp53 suivant cette même nomenclature. On utilise dans ce qui suit une autre nomenclature pour ces trois protéines structurales, qui sera C pour p20, gE1 pour gp62 et gE2 pour gp53.

R.O. Donis et al. (Neutralizing Monoclonal Antibodies to Bovine Viral Diarrhoea Virus Bind to the 56 K to 58 K Glycoprotein - J. Gen. Virol. (1988), 69:77-86) ont montré la présence d'épitopes de neutralisation dans une protéine glycosylée d'enveloppe de 56-58 kD.

D'après la demande de brevet EP-A-0.236.977, on connaît également un épitope peptidique neutralisant qui s'avère être situé au niveau du site d'épissage entre les glycoprotéines gE1 et gE2.

La présente invention a pour objectif de mettre en évidence d'autres épitopes neutralisants et d'obtenir les peptides ou polypeptides les incluant et de réaliser des compositions vaccinales, y compris vaccins synthétiques et vaccins vivants recombinants, contre le virus de la diarrhée bovine et virus apparentés.

Un autre objectif de l'invention est de fournir de tels vaccins qui soient efficaces contre les deux biotypes du virus BVD.

Conformément à l'invention, on a localisé des épitopes neutralisants dans une région codée par la partie 5' du génome du virus BVD/Osloss et, à partir des informations de localisation, on a réalisé des constructions d'ADN qui comprennent une fraction du génome BVD, laquelle contient au moins une séquence de nucléotides correspondant à l'un au moins de ces épitopes. Plus précisément, les séquences relatives à ces épitopes ont été notamment localisées dans la première moitié de la partie du génome viral (transcrit en ADN) qui code pour gE2. On a aussi localisé des épitopes dans la séquence codant pour gE1.

L'invention a donc pour objet une séquence de nucléotides correspondant sensiblement à la fraction du génome des virus BVD codant pour gE2 ou séquence contenue à son intérieur, et codant pour les 273 premiers acides aminés de gE2, et à une séquence de nucléotides correspondant à la fraction des virus BVD codant pour gE1 ou séquence contenue à son intérieur, ainsi que toute nouvelle séquence nucléotidique qui les contient, séparément ou en combinaison, et qui comporte des moyens permettant leur expression ou qui soit

associée à de tels moyens.

Une séquence de nucléotides correspondant sensiblement aux 273 premiers acide aminés de la partie du génome, de la souche Osloss (B. Liess, 1967, Dtsch. Tierärtztl. Wochenschr., 74:46-49), codant pour gE2 est représentée, à titre d'exemple, dans la liste de séquences annexée, sous la référence SEQ ID NO:1. Pour cette même souche, une séquence codant pour gE1 est représentée sous la référence SEQ ID NO:3.

A titre d'exemple, de telles séquences peuvent être les suivantes :

- codon d'initiation de traduction - séquence codant pour gE1 - séquence codant pour gE2 - signal de fin de copie (une séquence complète est représentée à titre d'exemple dans la liste de séquences sous la référence SEQ ID NO:2) ;
- codon d'initiation de traduction - séquence codant pour gE1 + les 273 premiers codons de gE2 - signal de fin de copie (par exemple SEQ ID No:4) ;
- codon d'initiation de traduction - séquence codant pour la fin de gE1 + les 273 premiers codons de gE2 - signal de fin de copie ;
- codon d'initiation de traduction - séquence codant pour les 273 premiers codons de gE2 - signal de fin de copie ;
- codon d'initiation de traduction - séquence codant pour gE2 - signal de fin de copie ;
- codon d'initiation de traduction - séquence codant pour la première moitié de gE2 - signal de fin de copie ;
- codon d'initiation de traduction - séquence codant pour C - séquence codant pour gE1 - séquence codant pour gE2 - signal de fin de copie.

L'invention a également pour objet les fragments nucléotidiques appartenant à cette première moitié de ladite partie du génome codant pour gE2, ou à la partie codant pour gE1, et dont les produits de traduction sont reconnus par des anticorps neutralisants, lesdits fragments étant associés à des moyens permettant leur expression.

Bien entendu, les séquences de nucléotides précitées incluent toutes les séquences équivalentes, c'est-à-dire qui reprennent les propriétés essentielles de la séquence. A titre d'exemple, ce serait le cas d'une séquence qui code pour une séquence identique d'acides aminés, mais qui utiliserait d'autres codons spécifiques par dégénérescence du code.

Ce serait également le cas pour une séquence codant pour une séquence d'acides aminés non plus identique mais similaire, compte tenu des ressemblances entre acides aminés.

Bien entendu, les séquences peuvent être issues de différentes souches de Pestivirus ou être constituées de séquences hybrides résultant de l'assemblage de fragments génomiques issus de souches différentes. Les séquences précitées incluent donc toute séquence différente conservant la reconnaissance par les mêmes anticorps neutralisants.

Ces séquences peuvent être des séquences d'ARN ou d'ADN, orientées positivement dans le sens 5', 3'.

L'invention a également pour objet les peptides ou polypeptides correspondant à la traduction de ces séquences de nucléotides.

Ces peptides ou polypeptides peuvent être préparés par différents procédés qui font également partie de l'invention.

Ainsi, dans un premier mode de mise en oeuvre, les séquences de nucléotides pertinentes selon l'invention peuvent être introduites dans des vecteurs d'expression viraux où eucaryotes.

Différentes techniques connues de construction de ces vecteurs sont décrites dans la demande de brevet EP-A-0.208.672.

Comme vecteurs d'expression viraux, on peut notamment utiliser le système Baculovirus (US-A-4.745.051) en intégrant la séquence nucléotidique concernée dans le génome de Baculovirus. Un exemple de construction dans ce système sera décrit plus loin.

Les hôtes eucaryotes peuvent être des cultures cellulaires animales ou des levures, notamment Saccharomyces cerevisiae. Les vecteurs de transfert pour les levures comportent avantageusement des marqueurs permettant la sélection des recombinants utiles, par exemple par résistance aux antibiotiques ou autres moyens de sélection connus (Broach J. et al., Meth. Enz. (1983) 101: 307). Pour les promoteurs, voir également Hess et al., J. Adv. Enz. Reg. (1968) 7:149, et Holland et al., Biochemistry (1968) 17:4900 ou Itzeman et al., J. Biol. Chem. (1980) 255:2073.

Les cellules animales sont, de préférence, des lignées cellulaires de mammifères connues telles que HeLa, CHO ou BHK, des cellules d'insectes, par exemple Spodoptera frugiperda (dépôt ATCC CRL 1711, Sf9) (notamment pour le système Baculovirus) et, d'une façon générale, on préférera des lignées dont l'utilisation pour l'expression de substances à administrer à l'animal a été reconnue par les autorités sanitaires. On utilisera comme promoteur, dans ces constructions cellulaires, des promoteurs viraux tels que ceux du virus SV40 (Fiers et al., Nature, (1978) 273:113) et du virus CMV ou cytomegalovirus humain (McGregor et Caskey, Nucleic Acids Res. 17:2365, 1989), ou encore celui du gène polyhédrine du Baculovirus AcNPV ou Autographa californica

nuclear polyhedrosis virus (Hooft van Iddekinge et al., 1983, Virology 131 : 561-565).

L'invention a donc également pour objet les hôtes eucaryotes permettant l'expression de ces peptides ou polypeptides, de préférence dans un état glycosylé.

Les peptides ou polypeptides selon l'invention peuvent également être fabriqués par synthèse peptidique usuelle (voir par exemple Stewart et al., Solid-Phase Peptide Synthesis, W. H. Freeman Co, San Francisco (1969) ou Merrifield, J. Am. Chem. Soc. 85:2149-2154 (1963) ou les brevets US-A-3.862.925, 3.842.047, 3.972.859 et 4.105.602).

Pour fabriquer rapidement de faibles quantités de peptides destinés par exemple à la recherche de réactions immunologiques avec des anticorps, monoclonaux ou non, on peut également utiliser une synthèse in vitro par traduction en système acellulaire (lysat de réticulocytes de lapin) additionné ou non de membranes microsomiales selon que l'on souhaite obtenir un peptide glycosylé ou non.

L'invention a également pour objet les vaccins incluant, dans une composition vaccinante efficace, ou exprimant, de tels peptides ou polypeptides, notamment les peptides issus d'une séquence codant pour gE1 et gE2 ou gE1 et la première moitié de gE2. Les peptides ou polypeptides peuvent être ou non conjugués à des supports et les vaccins peuvent présenter des adjuvants usuels.

Ces virus recombinants peuvent aussi être utilisés pour l'expression de peptides ou polypeptides selon l'invention dans des cultures de virus sur cellules.

Brève description du dessin.

La figure 1 montre, sur la carte du génome de BVD Osloss, la position des clones d'ADNc 63, 36 et 183 couvrant la région codant pour les protéines structurales de BVD, les sites de restriction utilisés ci-après pour le clonage du fragment gE1, la phase de lecture ouverte et les protéines structurales E1 et E2 ;

la figure 2 montre le profil d'hydrophobicité de la phase de lecture ouverte du génome de BVD Osloss codant pour les protéines structurales et la localisation des sites potentiels de glycosylation et des résidus cystéine sur la phase de lecture ouverte ;

la figure 3 montre la position de l'ensemble des sites de restriction utilisés ci-après ainsi que la stratégie suivie pour la préparation du fragment E1/830 ;

la figure 4 montre la localisation des trois principaux fragments, E1, E1/830 et E1/E2, dont le clonage est décrit ci-après, dans la partie du génome de BVD Osloss codant pour les protéines structurales ;

la figure 5 montre la carte de restriction du vecteur de transfert pJVP10Z ; les promoteurs du gène P10 et du gène de la polyhédrine (PH) ainsi que le sens de la transcription qu'ils initient sont indiqués par des flèches ; S indique le site de clonage ; et

la figure 6 montre la cinétique d'expression des glycoprotéines dans les cellules Sf9 infectées par AcNPV-E1/E2.

la figure 7 montre la partie du génome BVD Osloss avec la position des amplimères pour une construction incorporant les séquences C, gE1, gE2.

1. Localisation des régions codant pour les glycoprotéines.

A - Localisation de la région codant pour gE1.

On a représenté sur la figure 1 la partie du génome viral relative aux protéines membranaires. La protéine gE1 (protéine glycosylée de 56-58 kD ; non glycosylée de 48 kD) est une protéine membranaire du virus BVD. Les données suivantes ont permis sa localisation :

a) Une partie de la phase de lecture ouverte est très riche en sites de glycosylation potentiels et en cystéine, ce qui est typique de protéines membranaires glycosylées (voir figure 2 ).

b) Un sérum de lapin dirigé contre la protéine fusion β-gal-C63 diminue la taille des plages de lyse en reconnaissant vraisemblablement des épitopes à la surface du virus.

c) Le début de la protéine gE1 a été déterminé par la présence d'une séquence signal caractéristique (von Heijne G., N.A.R. 14:4683-4690, 1986) débutant au niveau de l'acide aminé 250 et précédée d'un tripeptide Arg-Lys-Lys pouvant être le signal d'épissage de la première protéine (voir figure 2).

d) La fin de la protéine a été déterminée d'après la taille attendue pour la protéine non glycosylée. Elle coïncide avec la localisation d'une hélice potentiellement membranaire.

En résumé, la taille de la protéine gE1 non glycosylée sera :
- avec la séquence signal (non clivée) : 453 acides aminés ;
- sans la séquence signal : 425 acides aminés.

B - Localisation de la région codant pour gE2.

Cette région contient 4 sites de glycosylation potentiels (voir figure 2) ; la fin de la protéine a été déterminée d'une part d'après la taille attendue pour la protéine non glycosylée et d'autre part sur la base de l'analyse du profil d'hydrophobicité, la séquence codant pour gE2 étant suivie d'une région fortement hydrophobe, hautement conservée parmi les Pestivirus connus et dont le rôle est encore mal identifié.

2. Préparation du fragment E1/830.

A - Clonage de la séquence codant pour gE1.

La séquence codant pour gE1 a été isolée à partir du clone 63 (C63) de la bibliothèque d'ADNc décrite dans la demande de brevet EP-A-0.208.672 (le clone y est désigné par pCT63), dans le plasmide pSP65 (vecteur décrit par Melton D.A. et al., 1984, dans Nucleic Acids Res. 12:7035-7056). Des clones de ce genre peuvent facilement être obtenus soit lors de la constitution d'une bibliothèque d'ADNc à partir de l'ARN viral comme cela est décrit dans la demande de brevet EP-A-0.208.672, soit par synthèse, soit encore par amplification (Polymerase Chain Reaction) à partir d'amplimères choisis dans la séquence donnée dans la susdite demande de brevet. Cette étape a été réalisée comme suit :

a) A partir du clone 63, on a isolé un fragment [BglI - BanII] contenant la quasi-totalité de la séquence codant pour gE1 (voir figure 1).

b) On a synthétisé un oligonucléotide synthétique dont la séquence comprend :

- une extrémité 5' EcoRI ;
- un codon ATG d'initiation de la traduction ;
- les 5 premiers codons de la séquence de gE1 ;
- un site de restriction BglI ;
- un site de restriction BanII ;
- les 6 derniers codons de la séquence de gE1 ;
- un codon TAG de terminaison de la traduction ;
- une extrémité 3' BamHI.

c) Cet oligonucléotide synthétique a été cloné entre les sites EcoRI et BamHI du plasmide pBR322 (Bolivar et al. 1977, Gene 2 : 95-113) préalablement délété de ses sites BglI et BanII internes ; ensuite, le fragment [BglI - BanII] isolé en a) a été inséré entre les sites correspondants de l'oligonucléotide cloné dans pBR322.
Le fragment résultant suit le schéma suivant :
[EcoRI - ATG - Séquence codant pour gE1 - codon STOP-BamHI]

Sa taille est de 1376 paires de bases (pb). Sa séquence nucléotidique est référencée SEQ ID NO:3 dans la liste de séquences annexée.

d) Ce fragment [EcoRI - BamHI] a ensuite été transféré dans le plasmide pSP65 qui contient le promoteur pour l'ARN polymérase du bactériophage SP6, pour donner le plasmide pSP65-gE1 et permet ainsi la transcription in vitro de la séquence insérée.

B - Clonage d'une séquence codant pour une partie de gE2 dans le plasmide pSP65-gE1.

On a utilisé le clone 36 (C36), de la susdite bibliothèque (pCT36), dont la position sur la carte est représentée sur la figure 1.

A partir de ce clone 36, on a isolé, par les enzymes de restriction, un fragment PvuII-NdeI comprenant la fin de la séquence codant pour gE1 et 273 codons de la séquence codant pour gE2. La position des sites de restriction ainsi que la stratégie suivie sont illustrées à la figure 3.

On a synthétisé un oligonucléotide comprenant :

- une extrémité 5' NdeI,
- un codon TAG de terminaison de la traduction,
- une extrémité 3' BamHI.

Cet oligonucléotide a été ajouté au fragment PvuII-NdeI.

Le fragment résultant a ensuite été digéré par BglII et BamHI et inséré dans le plasmide pSP65-gE1 décrit en A.

Le fragment final suit donc le schéma suivant :
[EcoRI - ATG - séquence codant pour gE1 - 830 nucléotides de gE2 - STOP - BamHI]. Sa taille est de 2200 pb. Sa séquence nucléotidique est donnée dans la liste de séquences sous la référence SEQ ID NO:4.

3. Transcription/traduction in vitro.

Le fragment obtenu en 2.B-, cloné dans le plasmide pSP65, a été transcrit in vitro, puis traduit dans un système acellulaire (lysat de réticulocytes de lapin). En fait, deux traductions ont été faites, l'une en présence de membranes microsomiales de pancréas de chien, l'autre en l'absence de membranes microsomiales.

L'analyse du produit de traduction montre, en l'absence de membranes microsomiales, un poids moléculaire estimé à 80 000 daltons.

Lorsque la traduction est faite en présence des membranes microsomiales qui conduisent à la maturation et à la glycosylation de la protéine, on obtient une première protéine glycosylée de 62 kD et une deuxième protéine de 40 kD environ. La protéine de 62 kD est la protéine gE1 comme le montrent des expériences similaires réalisées à partir du clone 63 de la bibliothèque d'ADNc. La protéine de 40 kD environ correspond au début de la protéine gE2.

4. Immunoprécipitation des produits de traduction.

Les différentes protéines synthétisées ont été immunoprécipitées à l'aide de deux types d'anticorps monoclonaux neutralisants : la protéine glycosylée de 62 kD est reconnue par le premier groupe, tandis que la protéine de 40 kD correspondant à gE2 contient au moins un épitope reconnu par le deuxième groupe de monoclonaux.

Tous les anticorps immunoprécipitent également la protéine de 80 kD observée en l'absence de membranes microsomiales, ce qui montre que la glycosylation n'est pas indispensable à la reconnaissance par ces anticorps.

Les vérifications effectuées avec les constructions aboutissant à gE1 ne conduisent à une immunoprécipitation qu'en présence du premier groupe d'anticorps, de même que les expériences conduites avec une construction incorporant gE1 prolongée des 32 premiers codons de gE2 et présentant donc l'épitope décrit dans la demande de brevet EP-A-0.236.977.

5. Préparation du fragment E1/E2.

Les sites de restriction StuI et AvaI utilisés pour prolonger le fragment E1/830 et construire le fragment complet E1/E2 sont indiqués sur la figure 3. La stratégie suivie a été la suivante :

a) Isolement du fragment [StuI-AvaI] à partir du clone 183 issu de la bibliothèque d'ADNc (où il est désigné par pCT183) identifiée ci-dessus (C183 à la figure 3).

b) Synthèse d'un oligonucléotide dont la séquence comprend une extrémité 5' StuI, un site de restriction AvaI, un codon TGA de terminaison de la traduction et une extrémité 3' BamHI.

c) Clonage de l'oligonucléotide entre les sites de restriction StuI et BamHI du plasmide pSP65 contenant le fragment E1/830. Le site StuI de ce plasmide est situé dans le fragment E1/830, 361 nucléotides en amont du site NdeI utilisé dans la préparation de E1/830, et le site BamHI correspond à l'extrémité 3' du fragment cloné dans pSP65 (voir figure 3).

d) Digestion du plasmide résultant par StuI et AvaI.

e) Clonage du fragment isolé en a) dans le plasmide obtenu en d).

Suivant cette procédure, le fragment E1/830 a été prolongé d'une longueur de 277 nucléotides ; le fragment résultant est long de 2487 nucléotides et comprend les séquences complètes codant pour les deux glycoprotéines E1 et E2. Ce fragment E1/E2 suit le schéma suivant :

[EcoRI - ATG - séquence codant pour E1 - séquence codant pour E2 - TGA - BamHI]. Sa localisation est illustrée à la figure 4, par comparaison aux deux fragments E1 et E1/830.

Sa séquence est annexée ci-après : SEQ ID NO:2.

6. Expression dans le système Baculovirus.

L'intégration du fragment E1/E2 dans le génome du Baculovirus Autographa californica nuclear polyhedrosis virus (AcNPV) a été réalisée par l'intermédiaire du vecteur de transfert pJVP10Z (J. Vialard et al., 1990, Journal of Virology 64 : 37-50) dont la carte de restriction est illustrée à la figure 5.

Le vecteur pJVP10Z contient :

* un site de clonage NheI placé entre le promoteur et le site de polyadénylation du gène de la polyhédrine de AcNPV ;

* le gène de résistance à l'ampicilline et l'origine de réplication du plasmide pUC8 (J. Messing, 1983, Meth. Enzymol. 101 : 20) ;

* le gène lacZ codant pour la β-galactosidase, placé sous le contrôle du promoteur P10 de AcNPV et suivi du signal de polyadénylation du virus SV40. Le repérage des recombinants peut se faire plus facilement en ajoutant dans l'agarose de recouvrement un substrat indicateur pour la β-galactosidase, aboutissant à la coloration bleue des plages recombinantes.

Le fragment E1/E2 a été cloné dans le site de restriction NheI du plasmide pJVP10Z et le plasmide recombinant pJVP10Z-E1/E2 a été utilisé pour cotransfecter des cellules d'insectes (Sf9, cellules de Spodoptera frugiperda, voir J. Vialard et al. cité ci-dessus) en même temps que de l'ADN purifié de AcNPV sauvage. La purification d'un virus recombinant AcNPV-E1/E2 a ensuite été réalisée par étalement du surnageant de cotransfection en boîtes de Petri et isolement des plages virales par recouvrement d'agarose ; deux critères permettent de distinguer les plages recombinantes de celles provoquées par du virus sauvage : l'expression de la β-galactosidase (voir ci-dessus) et l'absence de polyhédrine nettement visible par contre dans les cellules infectées par le virus sauvage.

7. Mise en évidence des épitopes de neutralisation.

Les glycoprotéines exprimées dans le système Baculovirus peuvent être mises en évidence dans un ELISA sandwich faisant intervenir un mélange de deux anticorps monoclonaux neutralisants comme anticorps capteurs (12B1 et 204B11), et le monoclonal 23G11 non neutralisant, comme anticorps révélateur.

12B1 : anti- gE1
204B11 : anti-gE2

Les deux anticorps capteurs étant dirigés contre des épitopes distincts, leur réactivité a été testée individuellement en réalisant un ELISA répondant au schéma suivant :

| Capteur | | Echantillon | | Révélateur |
|---|---|---|---|---|
| 12B1 ou 204B11 | - | Sf9/AcNPV-E1/E2 | - | 23G11* |

Les résultats de ce test sont rassemblés dans le tableau 1.

Tableau 1 : Réactivité en ELISA des deux monoclonaux neutralisants 12B1 et 204B11 sur les protéines exprimées par le virus recombinant AcNPV-E1/E2.

ELISA sandwich : capteurs = 12B1 ou 204B11 ; révélateur = 23G11* (dilution anticorps : 4000x).

Sf9/AcNPV-E1/E2 : cellules Sf9 (Spodoptera frugiperda) infectées par le recombinant AcNPV-E1/E2 et récoltées à 72 heures post-infection ; Sf9 : cellules non infectées.

Les valeurs sont exprimées en DO X 1000 (OVER = DO > 2).

| Capteur | Dilution des cellules | Sf9/AcNPV-E1/E2 | Sf9 |
|---|---|---|---|
| 12B1 | 3x | OVER | 72 |
| | 9x | 1558 | 49 |
| | 27x | 1078 | 44 |
| | 81x | 259 | 48 |
| 204B11 | 3x | OVER | 46 |
| | 9x | OVER | 36 |
| | 27x | 1915 | 32 |
| | 81x | 602 | 35 |

Ces résultats montrent que les protéines exprimées sont captées par les deux types d'anticorps neutralisants testés ; il est important de rappeler que ces deux monoclonaux diffèrent sous deux aspects :

a. des expériences de transcriptions/ traductions/immunoprécipitations ont déterminé qu'ils étaient dirigés contre des épitopes distincts situés l'un dans la glycoprotéine gE1, l'autre dans gE2 ; leur capacité à capter les protéines produites en Baculovirus montre par conséquent que non seulement les deux protéines sont exprimées, mais qu'elles le sont en outre dans une conformation permettant leur reconnaissance par les anticorps monoclonaux neutralisants.

b. les deux anticorps neutralisants ont été developpés à partir d'antigènes d'immunisation différents : le 204B11 (ou BD204B11) est issu de la souche BD/Moredun, le 12B1 (ou NY12B1) de la souche New York ; le 216E7 (ou AV216E7, anti-gE2), issu de la souche Aveyron, reconnaît également de manière spécifique les protéines exprimées en Baculovirus qui proviennent de la souche Osloss.

8. Cinétique d'expression des glycoprotéines.

Le niveau d'expression des glycoprotéines dans les cellules Sf9 infectées par le recombinant AcNPV-E1/E2 était encore en phase ascendante 72 heures après l'infection ; on a donc réalisé une cinétique d'infection s'étendant sur une période plus longue (11 jours), et le taux en glycoprotéines exprimées a été estimé au cours du temps d'après les valeurs obtenues en ELISA. Il s'agit d'un ELISA sandwich réalisé suivant le même schéma que celui décrit en 7, dans lequel les anticorps neutralisants 12B1 et 204B11 sont utilisés individuellement comme capteurs et le 23G11* comme révélateur. Les résultats sont illustrés sur la figure 6 :

204B11:A, B, C = courbes obtenues avec le monoclonal 204B11 utilisé comme capteur, et pour trois

dilutions de cellules Sf9 infectées (4x, 12x et 36x).

12B1:B, C = courbes obtenues avec le monoclonal 12B1 utilisé comme capteur, pour deux dilutions de cellules infectées (12x et 36x).

Le niveau d'expression des protéines reconnues en ELISA , quel que soit l'anticorps utilisé comme capteur, atteint un plateau 4 jours après l'infection (99 heures), plateau qui persiste pratiquement jusqu'au septième jour post-infection (167 heures). Le parallélisme observé entre les courbes obtenues avec les deux types de capteurs confirme la présence des deux épitopes dans les protéines exprimées, épitopes qui sont maintenus avec la même stabilité tout au long de la cinétique d'infection.

9. Localisation des protéines exprimées.

Les anticorps monoclonaux neutralisants 204B11, 12B1 et 216E7 ont été utilisés pour mettre en évidence les glycoprotéines par la technique d'immunofluorescence indirecte. Pour ce faire, les cellules Sf9 infectées par le recombinant AcNPV-E1/E2 ont été soumises à différentes conditions de fixation :

- dans de l'acétone 95 %, 20 minutes à -20°C ;
- dans du formol 4 %, 10 minutes à température ambiante ;
- 10 minutes dans du formol 4 %, suivi de 5 minutes dans du Triton X-100 0,1 %.

Les conditions de fixation à l'acétone sont trop drastiques pour permettre une localisation précise des protéines exprimées : les membranes sont perméabilisées et la fluorescence est intense mais répartie dans tout le cytoplasme. Par contre, lorsque les cellules sont fixées au formol, la fluorescence est visible sous forme de granules localisés sur toute la surface des cellules infectées.

Le fait d'ajouter à la fixation au formol une étape de traitement au Triton X-100 fragilise la membrane cytoplasmique sans perméabiliser les noyaux, ce qui amène une fluorescence plus intense que précédemment, granuleuse, entrant par endroits dans le cytoplasme.

Il est important de souligner que les deux anticorps 12B1 (NY ; anti- gE1) et 204B11 (BD ; anti-gE2) donnent des images tout à fait comparables, et qu'il en est de même pour le 216E7 (AV ; anti-gE2). Ces résultats confirment par conséquent la présence des deux glycoprotéines, qui sont exprimées à la surface des cellules infectées et qui pourraient être associées l'une à l'autre.

Les glycoprotéines exprimées sont reconnues par des anticorps monoclonaux neutralisants, qui diffèrent à deux niveaux. D'une part, ils sont dirigés contre des épitopes distincts situés l'un dans la première glycoprotéine, l'autre dans la deuxième ; les résultats montrent par conséquent que les deux protéines sont toutes deux présentes dans les cellules infectées par le recombinant et suivent une courbe d'expression tout à fait comparable. D'autre part, ils diffèrent au niveau des souches virales utilisées comme antigènes d'immunisation :

- 12B1 : souche BVD/New-York (Rhône Mérieux, France) ;
- 204B11 : souche BD/Moredun (Rhône Mérieux, France) ;
- 216E7 : souche Aveyron (Rhône Mérieux, France).

Cette constatation surprenante est intéressante dans le cadre de l'utilisation des glycoprotéines exprimées (issues de la souche Osloss) à des fins de vaccination, étant donné la reconnaissance comparable de ces protéines par des anticorps issus de souches virales sérologiquement aussi éloignées que Aveyron et New-York. Il est à noter par exemple que l'anticorps 216E7 non seulement ne neutralise pas le virus New-York, mais ne le reconnaît pas en immunofluorescence.

Enfin, les images observées en immunofluorescence dans différentes conditions de fixation des cellules montrent clairement que les glycoprotéines, révélées par les anticorps monoclonaux neutralisants, sont exprimées à la surface des cellules infectées.

Il est à noter que les glycoprotéines gE1 et gE2 sont exprimées à un niveau suffisant pour être observables en gel d'électrophorèse après coloration au bleu de Coomassie. Leurs positions sur gel correspondent aux poids moléculaires attendus après clivage et glycosylation, ce qui laisse supposer une maturation correcte des protéines exprimées dans ce système.

10. E1/E2 : Immunisation de lapins et analyse des anticorps induits.

Un lysat de cellules d'insectes Sf9 (Spodoptera frugiperda) infectées par le recombinant AcNPV-E1/E2 a été utilisé dans des essais d'immunisation de lapins réalisés suivant le protocole suivant :

Matériel :

- antigène d'immunisation : cellules Sf9 infectées par AcNPV-E1/E2 ; antigène témoin : cellules Sf9 non infectées ;
- 6 lapins : 3 doses d'antigène, 1 témoin pour chaque dose: Lapins n° 1, 2, 3 : inoculation de cellules infectées, à raison, respectivement, de $20.10^6$, $4.10^6$ et $0,8.10^6$ cellules par inoculation.

Lapins n° 4, 5, 6 : inoculation de cellules témoins, à raison, respectivement, de $20.10^6$, $4.10^6$ et $0,8.10^6$ cellules par inoculation.

Immunisation :

- jour 0 : 1ère inoculation ;
- jour 28 : 2ème inoculation ;
- jour 56 : 3ème inoculation ;
- jour 85 : 4ème inoculation ;
- jour 110 : 5ème inoculation.

Analyse sérologique :

Les sérums ont été prélevés avant la première inoculation, puis aux jours 38, 66, 99 et 120. Les anticorps BVD-spécifiques sont ensuite analysés en ELISA compétition vis-à-vis d'anticorps monoclonaux neutralisants, ainsi que par séroneutralisation contre trois souches virales : Aveyron, New-York et Osloss non cytopathogène.

Nomenclature utilisée pour les prélèvements successifs :

| N° lapin | Dose inoculée | Prélèvements Jour 38 | Jour 66 | Jour 99 | Jour 120 |
|---|---|---|---|---|---|
| 1 | $20.10^6$ CI | S1/P38 | S1/P66 | S1/P99 | S1/P120 |
| 2 | $4.10^6$ CI | S2/P38 | S2/P66 | S2/P99 | S2/P120 |
| 3 | $0,8.10^6$ CI | S3/P38 | S3/P66 | S3/P99 | S3/P120 |
| 4 | $20.10^6$ CTe | S4/P38 | S4/P66 | S4/P99 | S4/P120 |
| 5 | $4.10^6$ CTe | S5/P38 | S5/P66 | S5/P99 | S5/P120 |
| 6 | $0,8.10^6$ CTe | S6/P38 | S6/P66 | S6/P99 | S6/P120 |

CI = cellules Sf9 infectées par AcNPV-E1/E2 ;

CTe = cellules Sf9 non infectées.

a) Résultats obtenus en ELISA compétition.

Les sérums prélevés après les 2ème et 3ème inoculations de lysats cellulaires bruts (P38 et P66) ont été analysés pour leur capacité à entrer en compétition avec un anticorps monoclonal neutralisant, en l'occurrence BD204B11, dans sa liaison avec l'antigène E1/E2 produit dans les cellules d'insectes. Les résultats sont rassemblés dans le tableau 2.

A partir de ces valeurs, on a calculé le pourcentage de compétition des sérums 1 à 3 (antigène CI) par rapport à la DO de base (témoin sans sérum), après correction en fonction de leurs contrôles respectifs par les lapins 4 à 6 (antigène CTe), autrement dit :

$$\%age\ compétition = \frac{(DO\ CTe - DO\ CI)}{DO\ TSS} \times 100 \qquad (I)$$

où CI = cellules Sf9 infectées par AcNPV-E1/E2 ;
CTe = cellules Sf9 non infectées ;
TSS = témoin sans sérum.

Les valeurs calculées sont reprises dans le tableau 3.

Tableau 2 : Réactivité en ELISA des sérums prélevés sur les 6 lapins aux jours 38 et 66.

Fixation de l'antigène E1/E2 par l'intermédiaire de l'anticorps NY12B1 (anti-gE1) utilisé comme capteur (ascite dilué 4000x). Anticorps compétiteur : BD204B11 (anti-gE2 ; monoclonal purifié et marqué, utilisé à la dilution 1000x). Les valeurs sont exprimées en DOx1000. La nomenclature des échantillons est celle décrite ci-dessus.

---

Dilution des prélèvements

| | 375x | 75x | 15x |
|---|---|---|---|
| 1ère série de prélèvements (P38) : | | | |
| Dose : $20.10^6$ cel./inj. | | | |
| S1 (CI) | 1076 | 936 | 727 |
| S4 (CTe) | 965 | 903 | 780 |
| Dose : $4.10^6$ cel./inj. | | | |
| S2 (CI) | 971 | 812 | 555 |
| S5 (CTe) | 993 | 847 | 597 |
| Dose : $0,8.10^6$ cel./inj. | | | |
| S3 (CI) | 1006 | 822 | 608 |
| S6 (CTe) | 929 | 809 | 571 |
| 2ème série de prélèvements (P66) : | | | |
| Dose : $20.10^6$ cel./inj. | | | |
| S1 (CI) | 1001 | 957 | 912 |
| S4 (CTe) | 1064 | 1014 | 897 |
| Dose : $4.10^6$ cel./inj. | | | |
| S2 (CI) | 973 | 773 | 406 |
| S5 (CTe) | 1083 | 957 | 800 |
| Dose : $0,8.10^6$ cel./inj. | | | |
| S3 (CI) | 1012 | 889 | 765 |
| S6 (CTe) | 1052 | 983 | 939 |

TSS (témoin sans sérum) : 1103

Tableau 3 : Taux de compétition des sérums prélevés aux jours 38 et 66, vis-à-vis du monoclonal BD204B1

(exprimés en % et calculés d'après les valeurs du tableau 2, suivant la formule (I).

| | | Dilution des prélèvements | | |
|---|---|---|---|---|
| | | 375x | 75x | 15x |
| P38 | S1 | 0 | 0 | 4,8 |
| | S2 | 2 | 3,2 | 3,8 |
| | S3 | 0 | 0 | 0 |
| P66 | S1 | 5,7 | 5,2 | 0 |
| | S2 | 10 | 16,7 | 35,7 |
| | S3 | 0 | 8,5 | 15,7 |

b) Séroneutralisations.

On a testé la présence d'anticorps neutralisants dans les quatre séries de prélèvements P38, P66, P99 et P120, et ce vis-à-vis de trois souches de Pestivirus cultivées sur cellules OCK :

- Osloss non cytopathogène ;
- New York ;
- Aveyron.

Les résultats sont rassemblés dans le tableau 4.

Tableau 4 : Titres séroneutralisants de sérums prélevés aux jours 38, 66, 99 et 120, vis-à-vis des souches Osloss NC (OsNC), New York (NY) et Aveyron (AV) (100 ufp/cupule).

Les valeurs sont exprimées en dilution de sérum pour laquelle on obtient 50 % de neutralisation des virus testés.

0 : pas d'effet observable ;

| n° lapin | 1 | 2 | 3 | 4 | 5 | 6 |
|---|---|---|---|---|---|---|
| **Souche OsNC :** | | | | | | |
| - P38 | 4 | 16 | 16 | 0 | 0 | 0 |
| - P66 | 32 | 256 | 64 | 0 | 0 | 0 |
| - P99 | 4096 | 4096 | 4096 | 0 | 0 | 0 |
| - P120 | 16384 | 4096 | 16384 | 0 | 0 | 0 |
| **Souche NY :** | | | | | | |
| - P38 | 4 | 4 | 4 | 0 | 0 | 0 |
| - P66 | 4 | 4 | 0 | 0 | 0 | 0 |
| - P99 | 1024 | 16 | 256 | 0 | 0 | 0 |
| - P120 | 1024 | 16 | 1024 | 0 | 0 | 0 |
| **Souche AV :** | | | | | | |
| - P38 | 0 | 8 | 4 | 0 | 0 | 0 |
| - P66 | 0 | 32 | 4 | 0 | 0 | 0 |
| - P99 | 1024 | 512 | 256 | 0 | 0 | 0 |
| - P120 | 256 | 1024 | 1024 | 0 | 0 | 0 |

c) Conclusions.

Les protéines virales exprimées en cellules d'insectes par le Baculovirus recombinant AcNPV-E1/E2 induisent chez le lapin l'apparition d'anticorps neutralisants :

- vis-à-vis des trois souches de Pestivirus testées, soit Osloss non cytopathogène, New York et Aveyron ;
- chez les trois lapins immunisés par des doses différentes d'antigène ;
- dès le premier prélèvement, soit après deux injections d'antigène, avec un titre séroneutralisant faible mais significatif par comparaison aux lapins témoins ; le titre augmente aux prélèvements suivants et devient très significatif à partir du troisième prélèvement, et ce vis-à-vis des trois souches virales.

Les anticorps induits chez le lapin par l'inoculation d'extraits de cellules exprimant les antigènes recombinants E1/E2 neutralisent par conséquent au moins trois souches très différentes de Pestivirus qui sont Osloss NC (BVD), New York (BVD) et Aveyron (maladie de Border).

11. Prolongation des constructions E1/E2.

La figure 7 montre la partie du génome BVD/Osloss codant pour les protéines structurales C, E1, E2, avec les limites présumées des trois séquences codantes et la localisation des constructions précédentes au niveau

des régions E1/E2.

A partir de RNA total isolé de cellules OCK infectées par la souche virale BVD/Osloss cytopathogène, la séquence codant pour C a été amplifiée par PCR en utilisant les deux amplimères suivants A et B.

Ampli A (brin +): localisé au niveau du codon d'initiation de la traduction, en position 384 dans le génome;

```
séquence 5'-GGAATTCCCATG GAG TTG ATT ACA ATT -3'
                 EcoRI  NcoI
```

Ampli B (brin -): localisé à 640 bp en aval du début de la séquence E1, en position 1760 dans le génome;

```
séquence 5' ACT TTC TAG AGA ATT GGT CAT -3'
                     XbaI
```

Le fragment amplifié suit donc le schéma suivant:
[extrémité EcoRI - ATG inclus dans NcoI - 750 bp codant pour C - 640 bp correspondant au début de E1 - extrémité XbaI].

Ce fragment a été inséré à l'extrémité 5' des trois constructions gE1, gE1/830 et gE1 1100, au niveau du site de restriction XbaI.

ANNEXE : LISTE DES SEQUENCES.

SEQ ID NO : 1

TYPE DE SEQUENCE : séquence nucléotidique

LONGUEUR DE LA SEQUENCE : 894 paires de bases

NOMBRE DE BRINS : simple

CONFIGURATION : linéaire

TYPE DE MOLECULE : ADNc pour ARN génomique

ORIGINE : BVD Osloss

REGION TRADUITE : 1-894

PROPRIETES : code pour la première moitié de la glycoprotéine structurale gE2

```
Gln Val Leu Gln Gly Ile Leu Trp Leu Ile Leu Ile Thr Gly Ala Gln    16
CAA GTG TTG CAA GGC ATA CTG TGG TTG ATA CTC ATA ACA GGG GCA CAA    48

Gly Leu Pro Val Cys Lys Pro Gly Phe Tyr Tyr Ala Ile Ala Lys Asn    32
GGG CTC CCA GTT TGC AAA CCC GGC TTT TAC TAC GCC ATA GCC AAA AAT    96

Asn Glu Ile Gly Pro Leu Gly Ala Thr Gly Leu Thr Thr Gln Trp Tyr    48
AAT GAG ATC GGC CCT CTT GGG GCT ACG GGC CTC ACC ACT CAG TGG TAT   144

Glu Tyr Ser Asp Gly Met Arg Leu Gln Asp Thr Gly Val Val Val Trp    64
GAA TAC TCG GAT GGG ATG CGG CTG CAG GAC ACG GGA GTT GTA GTG TGG   192

Cys Lys Gly Gly Glu Ile Lys Tyr Leu Ile Thr Cys Glu Arg Glu Ala    80
TGT AAA GGT GGA GAG ATC AAA TAT CTA ATT ACA TGT GAG AGG GAA GCC   240

Arg Tyr Leu Ala Ile Leu His Thr Arg Ala Leu Pro Thr Ser Val Val    96
AGG TAT CTG GCC ATT CTA CAC ACG AGA GCC CTG CCG ACG TCT GTA GTA   288

Phe Glu Lys Ile Ile Asp Gly Lys Glu Gln Glu Asp Val Val Glu Met   112
TTT GAA AAA ATC ATA GAT GGA AAA GAA CAA GAG GAC GTA GTG GAA ATG   336

Asp Asp Asn Phe Glu Leu Gly Leu Cys Pro Cys Asp Ala Lys Pro Leu   128
GAT GAT AAC TTT GAA CTC GGT CTT TGC CCG TGT GAT GCT AAA CCC TTG   384

Val Arg Gly Lys Phe Asn Thr Thr Leu Leu Asn Gly Pro Ala Phe Gln   144
GTA AGG GGA AAA TTT AAT ACA ACA CTT CTG AAT GGG CCA GCC TTC CAG   432

Met Val Cys Pro Ile Gly Trp Thr Gly Thr Val Ser Leu Cys His Trp   160
ATG GTT TGC CCT ATA GGA TGG ACA GGA ACT GTG AGT CTG TGT CAC TGG   480

Ser Asn Lys Asp Thr Leu Ala Met Thr Val Val Arg Thr Tyr Lys Arg   176
TCC AAT AAG GAT ACG TTA GCC ATG ACC GTT GTA CGA ACA TAC AAG AGG   528

His Arg Pro Phe Pro Phe Arg Gln Gly Cys Ile Thr Gln Lys Val Ile   192
CAC AGG CCT TTC CCC TTT AGG CAA GGC TGC ATT ACC CAG AAA GTC ATC   576

Gly Gly Asp Leu Tyr Asp Cys Ala Leu Gly Gly Asn Trp Thr Cys Val   208
GGG GGA GAC CTC TAC GAC TGT GCC TTG GGA GGG AAC TGG ACT TGT GTA   624
```

| | | | | | | | | | | | | | | | | |
|---|---|---|---|---|---|---|---|---|---|---|---|---|---|---|---|---|
| Pro | Gly | Asp | Ile | Leu | Arg | Tyr | Val | Asp | Gly | Pro | Val | Glu | Ser | Cys | Lys | 224 |
| CCG | GGG | GAC | ATA | CTA | CGA | TAT | GTA | GAT | GGG | CCT | GTC | GAG | TCT | TGC | AAG | 672 |
| Trp | Cys | Gly | Tyr | Lys | Phe | His | Lys | Ser | Glu | Gly | Leu | Pro | His | Phe | Pro | 240 |
| TGG | TGT | GGT | TAC | AAG | TTT | CAT | AAA | AGT | GAG | GGT | CTG | CCA | CAC | TTC | CCA | 720 |
| Ile | Gly | Lys | Cys | Lys | Leu | Lys | Asn | Glu | Ser | Gly | Tyr | Arg | Gln | Val | Asp | 256 |
| ATT | GGC | AAG | TGC | AAG | CTG | AAG | AAT | GAA | AGT | GGC | TAC | AGA | CAA | GTA | GAT | 768 |
| Glu | Thr | Ser | Cys | Asn | Arg | Asp | Gly | Val | Ala | Ile | Val | Pro | Thr | Gly | Ser | 272 |
| GAG | ACC | TCT | TGC | AAC | AGA | GAC | GGT | GTG | GCT | ATA | GTA | CCA | ACT | GGT | TCG | 816 |
| Val | Lys | Cys | Lys | Ile | Gly | Asp | Thr | Val | Val | Gln | Val | Ile | Ala | Met | Asp | 288 |
| GTG | AAA | TGC | AAG | ATA | GGG | GAC | ACA | GTG | GTG | CAA | GTC | ATA | GCA | ATG | GAT | 864 |
| Asp | Lys | Leu | Gly | Pro | Met | Pro | Cys | Arg | Pro | | | | | | | 299 |
| GAT | AAG | CTA | GGG | CCT | ATG | CCT | TGC | AGA | CCA | | | | | | | 894 |

SEQ ID NO : 2

TYPE DE SEQUENCE : séquence nucléotidique

LONGUEUR DE LA SEQUENCE : 2487 paires de bases

NOMBRE DE BRINS : simple

CONFIGURATION : linéaire

TYPE DE MOLECULE : ADNc pour ARN génomique

ORIGINE : BVD Osloss

REGION TRADUITE : 10-2479

PROPRIETES : code pour les glycoprotéines structurales gE1 et gE2.

```
                Met Gly Lys Leu Glu Lys Ala Leu Leu Ala Trp Ala Val    13
GAA TTC ACC ATG GGC AAA CTA GAG AAA GCC CTG TTG GCA TGG GCA GTA        48
 EcoRI    NcoI
Ile Ala Leu Val Leu Phe Gln Val Ala Val Gly Glu Asn Ile Thr Gln        29
ATA GCC TTG GTT TTG TTT CAA GTC GCA GTG GGA GAG AAC ATA ACA CAA        96

Trp Asn Leu Gln Asp Asn Gly Thr Glu Gly Ile Gln Arg Ala Met Phe        45
TGG AAC TTA CAA GAC AAT GGG ACG GAA GGA ATA CAA CGG GCC ATG TTC       144

Gln Arg Gly Val Asn Arg Ser Leu His Gly Ile Trp Pro Glu Lys Ile        61
CAA AGA GGC GTA AAT AGA AGT CTG CAT GGG ATC TGG CCA GAG AAA ATC       192

Cys Thr Gly Val Pro Ser His Leu Ala Thr Asp Thr Glu Leu Lys Ala        77
TGT ACA GGT GTC CCC TCC CAC TTG GCC ACT GAT ACA GAA CTG AAG GCA       240

Ile His Gly Met Met Asp Ala Ser Glu Lys Thr Asn Tyr Thr Cys Cys        93
ATT CAT GGT ATG ATG GAT GCT AGC GAG AAG ACA AAT TAC ACA TGC TGC       288

Arg Leu Gln Arg His Glu Trp Asn Lys His Gly Trp Cys Asn Trp Tyr       109
AGG CTC CAA CGC CAT GAG TGG AAC AAG CAT GGT TGG TGC AAT TGG TAC       336

Asn Ile Glu Pro Trp Ile Val Leu Met Asn Lys Thr Gln Ala Asn Leu       125
AAT ATT GAA CCT TGG ATT GTT CTC ATG AAT AAA ACC CAA GCC AAC CTT       384

Ala Glu Gly Gln Pro Pro Arg Glu Cys Ala Val Thr Cys Arg Tyr Asp       141
GCT GAG GGT CAG CCA CCA AGG GAG TGT GCC GTT ACA TGC CGG TAT GAC       432

Arg Asp Ser Asp Leu Asn Val Val Thr Gln Ala Arg Asn Ser Pro Thr       157
CGA GAT AGT GAC CTA AAT GTA GTA ACA CAA GCT AGG AAC AGC CCC ACA       480

Pro Leu Thr Gly Cys Lys Lys Gly Lys Asn Phe Ser Phe Ala Gly Val       173
CCA TTG ACA GGC TGC AAG AAA GGC AAG AAC TTC TCC TTT GCA GGT GTG       528

Leu Val Gln Gly Pro Cys Asn Phe Glu Ile Ala Val Ser Asp Val Leu       189
TTG GTA CAA GGG CCT TGC AAC TTT GAA ATA GCT GTA AGT GAT GTG CTG       576

Phe Arg Glu His Asp Cys Thr Ser Val Ile Gln Gly Thr Ala His Tyr       205
TTT AGA GAG CAC GAT TGC ACA AGT GTG ATT CAA GGC ACG GCT CAC TAT       624
```

```
Leu Val Asp Gly Met Thr Asn Ser Leu Glu Ser Ala Arg Gln Gly Thr    221
CTG GTA GAC GGG ATG ACC AAT TCT CTA GAA AGT GCC AGG CAA GGG ACC    672

Ala Lys Leu Thr Thr Trp Leu Gly Arg Gln Leu Lys Lys Leu Gly Lys    237
GCA AAG TTA ACT ACT TGG TTG GGT AGG CAG CTT AAG AAA CTA GGG AAG    720

Lys Leu Glu Asn Lys Ser Lys Thr Trp Phe Gly Ala Tyr Ala Ala Ser    253
AAA CTG GAA AAC AAG AGT AAG ACA TGG TTT GGG GCA TAT GCA GCC TCT    768

Pro Tyr Cys Glu Val Glu Arg Arg Leu Gly Tyr Ile Trp Tyr Thr Lys    269
CCC TAC TGC GAG GTA GAA CGG AGG CTT GGT TAC ATC TGG TAT ACA AAG    816

Asn Cys Thr Pro Ala Cys Leu Pro Lys Asn Thr Lys Ile Val Gly Pro    285
AAT TGC ACC CCT GCC TGT TTA CCA AAA AAT ACA AAG ATC GTT GGC CCC    864

Gly Arg Phe Asp Thr Asn Ala Glu Asp Gly Lys Ile Leu His Glu Met    301
GGT AGG TTC GAC ACC AAT GCG GAG GAT GGT AAA ATA CTG CAT GAG ATG    912

Gly Gly His Leu Ser Glu Val Leu Leu Leu Ser Val Val Val Leu Ser    317
GGG GGC CAC TTG TCA GAG GTG CTA CTA CTC TCA GTG GTA GTG CTT TCC    960

Asp Phe Ala Pro Glu Thr Ala Ser Val Val Tyr Leu Ile Leu His Phe    333
GAT TTC GCT CCA GAG ACA GCC AGT GTG GTA TAT TTA ATT CTA CAT TTC   1008

Ser Ile Pro Gln Gly His Thr Asp Ile His Asp Cys Asp Lys Asn Gln    349
TCC ATC CCA CAA GGA CAC ACT GAC ATA CAT GAC TGT GAT AAA AAC CAA   1056

Leu Asn Leu Thr Val Gly Leu Thr Thr Ala Glu Val Ile Pro Gly Ser    365
CTA AAC CTC ACC GTA GGA CTC ACA ACA GCT GAA GTA ATA CCT GGG TCA   1104

Val Trp Asn Leu Gly Lys Tyr Val Cys Ile Arg Pro Asp Trp Trp Pro    381
GTT TGG AAT TTG GGC AAA TAT GTT TGT ATA AGA CCA GAT TGG TGG CCT   1152

Tyr Glu Thr Ala Thr Phe Leu Val Phe Glu Glu Val Gly Gln Val Ile    397
TAT GAG ACA GCC ACG TTC CTA GTG TTT GAA GAG GTG GGT CAA GTG ATC   1200

Arg Ile Val Leu Arg Ala Leu Arg Asp Leu Thr Arg Ile Trp Thr Ala    413
AGG ATA GTC TTG AGG GCT TTA AGA GAT CTA ACG CGC ATT TGG ACC GCT   1248

Ala Thr Thr Thr Ala Phe Leu Val Cys Leu Val Lys Val Val Arg Gly    429
GCT ACG ACT ACT GCA TTC CTG GTA TGT CTG GTG AAG GTG GTG AGA GGC   1296

Gln Val Leu Gln Gly Ile Leu Trp Leu Ile Leu Ile Thr Gly Ala Gln    445
CAA GTG TTG CAA GGC ATA CTG TGG TTG ATA CTC ATA ACA GGG GCA CAA   1344

Gly Leu Pro Val Cys Lys Pro Gly Phe Tyr Tyr Ala Ile Ala Lys Asn    461
GGG CTC CCA GTT TGC AAA CCC GGC TTT TAC TAC GCC ATA GCC AAA AAT   1392

Asn Glu Ile Gly Pro Leu Gly Ala Thr Gly Leu Thr Thr Gln Trp Tyr    477
AAT GAG ATC GGC CCT CTT GGG GCT ACG GGC CTC ACC ACT CAG TGG TAT   1440

Glu Tyr Ser Asp Gly Met Arg Leu Gln Asp Thr Gly Val Val Val Trp    493
GAA TAC TCG GAT GGG ATG CGG CTG CAG GAC ACG GGA GTT GTA GTG TGG   1488

Cys Lys Gly Gly Glu Ile Lys Tyr Leu Ile Thr Cys Glu Arg Glu Ala    509
TGT AAA GGT GGA GAG ATC AAA TAT CTA ATT ACA TGT GAG AGG GAA GCC   1536
```

```
Arg Tyr Leu Ala Ile Leu His Thr Arg Ala Leu Pro Thr Ser Val Val   525
AGG TAT CTG GCC ATT CTA CAC ACG AGA GCC CTG CCG ACG TCT GTA GTA  1584

Phe Glu Lys Ile Ile Asp Gly Lys Glu Gln Glu Asp Val Val Glu Met   541
TTT GAA AAA ATC ATA GAT GGA AAA GAA CAA GAG GAC GTA GTG GAA ATG  1632

Asp Asp Asn Phe Glu Leu Gly Leu Cys Pro Cys Asp Ala Lys Pro Leu   557
GAT GAT AAC TTT GAA CTC GGT CTT TGC CCG TGT GAT GCT AAA CCC TTG  1680

Val Arg Gly Lys Phe Asn Thr Thr Leu Leu Asn Gly Pro Ala Phe Gln   573
GTA AGG GGA AAA TTT AAT ACA ACA CTT CTG AAT GGG CCA GCC TTC CAG  1728

Met Val Cys Pro Ile Gly Trp Thr Gly Thr Val Ser Leu Cys His Trp   589
ATG GTT TGC CCT ATA GGA TGG ACA GGA ACT GTG AGT CTG TGT CAC TGG  1776

Ser Asn Lys Asp Thr Leu Ala Met Thr Val Val Arg Thr Tyr Lys Arg   605
TCC AAT AAG GAT ACG TTA GCC ATG ACC GTT GTA CGA ACA TAC AAG AGG  1824

His Arg Pro Phe Pro Phe Arg Gln Gly Cys Ile Thr Gln Lys Val Ile   621
CAC AGG CCT TTC CCC TTT AGG CAA GGC TGC ATT ACC CAG AAA GTC ATC  1872

Gly Gly Asp Leu Tyr Asp Cys Ala Leu Gly Gly Asn Trp Thr Cys Val   637
GGG GGA GAC CTC TAC GAC TGT GCC TTG GGA GGG AAC TGG ACT TGT GTA  1920

Pro Gly Asp Ile Leu Arg Tyr Val Asp Gly Pro Val Glu Ser Cys Lys   653
CCG GGG GAC ATA CTA CGA TAT GTA GAT GGG CCT GTC GAG TCT TGC AAG  1968

Trp Cys Gly Tyr Lys Phe His Lys Ser Glu Gly Leu Pro His Phe Pro   669
TGG TGT GGT TAC AAG TTT CAT AAA AGT GAG GGT CTG CCA CAC TTC CCA  2016

Ile Gly Lys Cys Lys Leu Lys Asn Glu Ser Gly Tyr Arg Gln Val Asp   685
ATT GGC AAG TGC AAG CTG AAG AAT GAA AGT GGC TAC AGA CAA GTA GAT  2064

Glu Thr Ser Cys Asn Arg Asp Gly Val Ala Ile Val Pro Thr Gly Ser   701
GAG ACC TCT TGC AAC AGA GAC GGT GTG GCT ATA GTA CCA ACT GGT TCG  2112

Val Lys Cys Lys Ile Gly Asp Thr Val Val Gln Val Ile Ala Met Asp   717
GTG AAA TGC AAG ATA GGG GAC ACA GTG GTG CAA GTC ATA GCA ATG GAT  2160

Asp Lys Leu Gly Pro Met Pro Cys Arg Pro Tyr Glu Ile Ile Pro Ser   733
GAT AAG CTA GGG CCT ATG CCT TGC AGA CCA TAT GAA ATC ATT CCC AGT  2208

Glu Gly Pro Val Glu Lys Thr Ala Cys Thr Phe Asn Tyr Thr Lys Thr   749
GAG GGG CCG GTA GAA AAG ACG GCA TGT ACC TTC AAC TAC ACA AAA ACA  2256

Leu Lys Asn Lys Tyr Tyr Glu Pro Arg Asp Asn Tyr Phe Gln Gln Tyr   765
TTA AAG AAC AAG TAT TAT GAG CCT AGG GAT AAT TAT TTC CAA CAA TAC  2304

Met Leu Lys Gly Glu Tyr Gln Tyr Trp Phe Asp Leu Glu Ile Thr Asp   781
ATG TTA AAA GGG GAG TAC CAA TAT TGG TTT GAC CTA GAG ATC ACT GAC  2352

His His Arg Asp Tyr Phe Ala Glu Ser Leu Leu Val Ile Val Val Ala   797
CAC CAC CGG GAT TAC TTC GCT GAG TCC CTA CTG GTG ATA GTG GTT GCA  2400

Leu Leu Gly Gly Arg Tyr Val Leu Trp Leu Leu Val Thr Tyr Met Ile   813
CTC CTG GGC GGT AGG TAC GTG CTC TGG TTA CTG GTT ACA TAT ATG ATC  2448

                                            STOP
Leu Ser Glu Gln Met Thr Ser Gly Gly Pro ***                       823
CTA TCA GAA CAA ATG ACC TCG GGA GGG CCC TGA GGA TCC              2487
                                            BamHI
```

SEQ ID NO : 3

TYPE DE SEQUENCE : séquence nucléotidique

LONGUEUR DE LA SEQUENCE : 1376 paires de bases

NOMBRE DE BRINS : simple

CONFIGURATION : linéaire

TYPE DE MOLECULE : ADNc pour ARN génomique

ORIGINE : BVD Osloss

REGION TRADUITE : 10-1371

PROPRIETES : code pour la glycoprotéine structurale gE1

```
         Met Gly Lys Leu Glu Lys Ala Leu Leu Ala Trp Ala Val     13
GAA TTC ACC ATG GGC AAA CTA GAG AAA GCC CTG TTG GCA TGG GCA GTA     48
EcoRI      NcoI
Ile Ala Leu Val Leu Phe Gln Val Ala Val Gly Glu Asn Ile Thr Gln     29
ATA GCC TTG GTT TTG TTT CAA GTC GCA GTG GGA GAG AAC ATA ACA CAA     96

Trp Asn Leu Gln Asp Asn Gly Thr Glu Gly Ile Gln Arg Ala Met Phe     45
TGG AAC TTA CAA GAC AAT GGG ACG GAA GGA ATA CAA CGG GCC ATG TTC    144

Gln Arg Gly Val Asn Arg Ser Leu His Gly Ile Trp Pro Glu Lys Ile     61
CAA AGA GGC GTA AAT AGA AGT CTG CAT GGG ATC TGG CCA GAG AAA ATC    192

Cys Thr Gly Val Pro Ser His Leu Ala Thr Asp Thr Glu Leu Lys Ala     77
TGT ACA GGT GTC CCC TCC CAC TTG GCC ACT GAT ACA GAA CTG AAG GCA    240

Ile His Gly Met Met Asp Ala Ser Glu Lys Thr Asn Tyr Thr Cys Cys     93
ATT CAT GGT ATG ATG GAT GCT AGC GAG AAG ACA AAT TAC ACA TGC TGC    288

Arg Leu Gln Arg His Glu Trp Asn Lys His Gly Trp Cys Asn Trp Tyr    109
AGG CTC CAA CGC CAT GAG TGG AAC AAG CAT GGT TGG TGC AAT TGG TAC    336

Asn Ile Glu Pro Trp Ile Val Leu Met Asn Lys Thr Gln Ala Asn Leu    125
AAT ATT GAA CCT TGG ATT GTT CTC ATG AAT AAA ACC CAA GCC AAC CTT    384

Ala Glu Gly Gln Pro Pro Arg Glu Cys Ala Val Thr Cys Arg Tyr Asp    141
GCT GAG GGT CAG CCA CCA AGG GAG TGT GCC GTT ACA TGC CGG TAT GAC    432

Arg Asp Ser Asp Leu Asn Val Val Thr Gln Ala Arg Asn Ser Pro Thr    157
CGA GAT AGT GAC CTA AAT GTA GTA ACA CAA GCT AGG AAC AGC CCC ACA    480

Pro Leu Thr Gly Cys Lys Lys Gly Lys Asn Phe Ser Phe Ala Gly Val    173
CCA TTG ACA GGC TGC AAG AAA GGC AAG AAC TTC TCC TTT GCA GGT GTG    528

Leu Val Gln Gly Pro Cys Asn Phe Glu Ile Ala Val Ser Asp Val Leu    189
TTG GTA CAA GGG CCT TGC AAC TTT GAA ATA GCT GTA AGT GAT GTG CTG    576

Phe Arg Glu His Asp Cys Thr Ser Val Ile Gln Gly Thr Ala His Tyr    205
TTT AGA GAG CAC GAT TGC ACA AGT GTG ATT CAA GGC ACG GCT CAC TAT    624
```

```
Leu Val Asp Gly Met Thr Asn Ser Leu Glu Ser Ala Arg Gln Gly Thr    221
CTG GTA GAC GGG ATG ACC AAT TCT CTA GAA AGT GCC AGG CAA GGG ACC    672

Ala Lys Leu Thr Thr Trp Leu Gly Arg Gln Leu Lys Lys Leu Gly Lys    237
GCA AAG TTA ACT ACT TGG TTG GGT AGG CAG CTT AAG AAA CTA GGG AAG    720

Lys Leu Glu Asn Lys Ser Lys Thr Trp Phe Gly Ala Tyr Ala Ala Ser    253
AAA CTG GAA AAC AAG AGT AAG ACA TGG TTT GGG GCA TAT GCA GCC TCT    768

Pro Tyr Cys Glu Val Glu Arg Arg Leu Gly Tyr Ile Trp Tyr Thr Lys    269
CCC TAC TGC GAG GTA GAA CGG AGG CTT GGT TAC ATC TGG TAT ACA AAG    816

Asn Cys Thr Pro Ala Cys Leu Pro Lys Asn Thr Lys Ile Val Gly Pro    285
AAT TGC ACC CCT GCC TGT TTA CCA AAA AAT ACA AAG ATC GTT GGC CCC    864

Gly Arg Phe Asp Thr Asn Ala Glu Asp Gly Lys Ile Leu His Glu Met    301
GGT AGG TTC GAC ACC AAT GCG GAG GAT GGT AAA ATA CTG CAT GAG ATG    912

Gly Gly His Leu Ser Glu Val Leu Leu Leu Ser Val Val Val Leu Ser    317
GGG GGC CAC TTG TCA GAG GTG CTA CTA CTC TCA GTG GTA GTG CTT TCC    960

Asp Phe Ala Pro Glu Thr Ala Ser Val Val Tyr Leu Ile Leu His Phe    333
GAT TTC GCT CCA GAG ACA GCC AGT GTG GTA TAT TTA ATT CTA CAT TTC   1008

Ser Ile Pro Gln Gly His Thr Asp Ile His Asp Cys Asp Lys Asn Gln    349
TCC ATC CCA CAA GGA CAC ACT GAC ATA CAT GAC TGT GAT AAA AAC CAA   1056

Leu Asn Leu Thr Val Gly Leu Thr Thr Ala Glu Val Ile Pro Gly Ser    365
CTA AAC CTC ACC GTA GGA CTC ACA ACA GCT GAA GTA ATA CCT GGG TCA   1104

Val Trp Asn Leu Gly Lys Tyr Val Cys Ile Arg Pro Asp Trp Trp Pro    381
GTT TGG AAT TTG GGC AAA TAT GTT TGT ATA AGA CCA GAT TGG TGG CCT   1152

Tyr Glu Thr Ala Thr Phe Leu Val Phe Glu Glu Val Gly Gln Val Ile    397
TAT GAG ACA GCC ACG TTC CTA GTG TTT GAA GAG GTG GGT CAA GTG ATC   1200

Arg Ile Val Leu Arg Ala Leu Arg Asp Leu Thr Arg Ile Trp Thr Ala    413
AGG ATA GTC TTG AGG GCT TTA AGA GAT CTA ACG CGC ATT TGG ACC GCT   1248

Ala Thr Thr Thr Ala Phe Leu Val Cys Leu Val Lys Val Val Arg Gly    429
GCT ACG ACT ACT GCA TTC CTG GTA TGT CTG GTG AAG GTG GTG AGA GGC   1296

Gln Val Leu Gln Gly Ile Leu Trp Leu Ile Leu Ile Thr Gly Ala Gln    445
CAA GTG TTG CAA GGC ATA CTG TGG TTG ATA CTC ATA ACA GGG GCA CAA   1344
                                STOP
Gly Leu Pro Val Cys Lys Pro Gly ***                                454
GGG CTC CCA GTT TGC AAA CCC GGC TAG GAT CC                        1376
                                    BamHI
```

SEQ ID NO : 4

TYPE DE SEQUENCE : séquence nucléotidique

LONGUEUR DE LA SEQUENCE : 2210 paires de bases

NOMBRE DE BRINS : simple

CONFIGURATION : linéaire

TYPE DE MOLECULE : ADNc pour ARN génomique

ORIGINE : BVD Osloss

REGION TRADUITE : 10-2206

PROPRIETES : code pour la glycoprotéine structurale gE1 et la première partie de la glycoprotéine structurale gE2

```
            Met Gly Lys Leu Glu Lys Ala Leu Leu Ala Trp Ala Val     13
GAA TTC ACC ATG GGC AAA CTA GAG AAA GCC CTG TTG GCA TGG GCA GTA     48

Ile Ala Leu Val Leu Phe Gln Val Ala Val Gly Glu Asn Ile Thr Gln     29
ATA GCC TTG GTT TTG TTT CAA GTC GCA GTG GGA GAG AAC ATA ACA CAA     96

Trp Asn Leu Gln Asp Asn Gly Thr Glu Gly Ile Gln Arg Ala Met Phe     45
TGG AAC TTA CAA GAC AAT GGG ACG GAA GGA ATA CAA CGG GCC ATG TTC    144

Gln Arg Gly Val Asn Arg Ser Leu His Gly Ile Trp Pro Glu Lys Ile     61
CAA AGA GGC GTA AAT AGA AGT CTG CAT GGG ATC TGG CCA GAG AAA ATC    192

Cys Thr Gly Val Pro Ser His Leu Ala Thr Asp Thr Glu Leu Lys Ala     77
TGT ACA GGT GTC CCC TCC CAC TTG GCC ACT GAT ACA GAA CTG AAG GCA    240

Ile His Gly Met Met Asp Ala Ser Glu Lys Thr Asn Tyr Thr Cys Cys     93
ATT CAT GGT ATG ATG GAT GCT AGC GAG AAG ACA AAT TAC ACA TGC TGC    288

Arg Leu Gln Arg His Glu Trp Asn Lys His Gly Trp Cys Asn Trp Tyr    109
AGG CTC CAA CGC CAT GAG TGG AAC AAG CAT GGT TGG TGC AAT TGG TAC    336

Asn Ile Glu Pro Trp Ile Val Leu Met Asn Lys Thr Gln Ala Asn Leu    125
AAT ATT GAA CCT TGG ATT GTT CTC ATG AAT AAA ACC CAA GCC AAC CTT    384

Ala Glu Gly Gln Pro Pro Arg Glu Cys Ala Val Thr Cys Arg Tyr Asp    141
GCT GAG GGT CAG CCA CCA AGG GAG TGT GCC GTT ACA TGC CGG TAT GAC    432

Arg Asp Ser Asp Leu Asn Val Val Thr Gln Ala Arg Asn Ser Pro Thr    157
CGA GAT AGT GAC CTA AAT GTA GTA ACA CAA GCT AGG AAC AGC CCC ACA    480

Pro Leu Thr Gly Cys Lys Lys Gly Lys Asn Phe Ser Phe Ala Gly Val    173
CCA TTG ACA GGC TGC AAG AAA GGC AAG AAC TTC TCC TTT GCA GGT GTG    528

Leu Val Gln Gly Pro Cys Asn Phe Glu Ile Ala Val Ser Asp Val Leu    189
TTG GTA CAA GGG CCT TGC AAC TTT GAA ATA GCT GTA AGT GAT GTG CTG    576

Phe Arg Glu His Asp Cys Thr Ser Val Ile Gln Gly Thr Ala His Tyr    205
TTT AGA GAG CAC GAT TGC ACA AGT GTG ATT CAA GGC ACG GCT CAC TAT    624
```

```
Leu Val Asp Gly Met Thr Asn Ser Leu Glu Ser Ala Arg Gln Gly Thr    221
CTG GTA GAC GGG ATG ACC AAT TCT CTA GAA AGT GCC AGG CAA GGG ACC    672

Ala Lys Leu Thr Thr Trp Leu Gly Arg Gln Leu Lys Lys Leu Gly Lys    237
GCA AAG TTA ACT ACT TGG TTG GGT AGG CAG CTT AAG AAA CTA GGG AAG    720

Lys Leu Glu Asn Lys Ser Lys Thr Trp Phe Gly Ala Tyr Ala Ala Ser    253
AAA CTG GAA AAC AAG AGT AAG ACA TGG TTT GGG GCA TAT GCA GCC TCT    768

Pro Tyr Cys Glu Val Glu Arg Arg Leu Gly Tyr Ile Trp Tyr Thr Lys    269
CCC TAC TGC GAG GTA GAA CGG AGG CTT GGT TAC ATC TGG TAT ACA AAG    816

Asn Cys Thr Pro Ala Cys Leu Pro Lys Asn Thr Lys Ile Val Gly Pro    285
AAT TGC ACC CCT GCC TGT TTA CCA AAA AAT ACA AAG ATC GTT GGC CCC    864

Gly Arg Phe Asp Thr Asn Ala Glu Asp Gly Lys Ile Leu His Glu Met    301
GGT AGG TTC GAC ACC AAT GCG GAG GAT GGT AAA ATA CTG CAT GAG ATG    912

Gly Gly His Leu Ser Glu Val Leu Leu Leu Ser Val Val Val Leu Ser    317
GGG GGC CAC TTG TCA GAG GTG CTA CTA CTC TCA GTG GTA GTG CTT TCC    960

Asp Phe Ala Pro Glu Thr Ala Ser Val Val Tyr Leu Ile Leu His Phe    333
GAT TTC GCT CCA GAG ACA GCC AGT GTG GTA TAT TTA ATT CTA CAT TTC   1008

Ser Ile Pro Gln Gly His Thr Asp Ile His Asp Cys Asp Lys Asn Gln    349
TCC ATC CCA CAA GGA CAC ACT GAC ATA CAT GAC TGT GAT AAA AAC CAA   1056

Leu Asn Leu Thr Val Gly Leu Thr Thr Ala Glu Val Ile Pro Gly Ser    365
CTA AAC CTC ACC GTA GGA CTC ACA ACA GCT GAA GTA ATA CCT GGG TCA   1104

Val Trp Asn Leu Gly Lys Tyr Val Cys Ile Arg Pro Asp Trp Trp Pro    381
GTT TGG AAT TTG GGC AAA TAT GTT TGT ATA AGA CCA GAT TGG TGG CCT   1152

Tyr Glu Thr Ala Thr Phe Leu Val Phe Glu Glu Val Gly Gln Val Ile    397
TAT GAG ACA GCC ACG TTC CTA GTG TTT GAA GAG GTG GGT CAA GTG ATC   1200

Arg Ile Val Leu Arg Ala Leu Arg Asp Leu Thr Arg Ile Trp Thr Ala    413
AGG ATA GTC TTG AGG GCT TTA AGA GAT CTA ACG CGC ATT TGG ACC GCT   1248

Ala Thr Thr Thr Ala Phe Leu Val Cys Leu Val Lys Val Val Arg Gly    429
GCT ACG ACT ACT GCA TTC CTG GTA TGT CTG GTG AAG GTG GTG AGA GGC   1296

Gln Val Leu Gln Gly Ile Leu Trp Leu Ile Leu Ile Thr Gly Ala Gln    445
CAA GTG TTG CAA GGC ATA CTG TGG TTG ATA CTC ATA ACA GGG GCA CAA   1344

Gly Leu Pro Val Cys Lys Pro Gly Phe Tyr Tyr Ala Ile Ala Lys Asn    461
GGG CTC CCA GTT TGC AAA CCC GGC TTT TAC TAC GCC ATA GCC AAA AAT   1392

Asn Glu Ile Gly Pro Leu Gly Ala Thr Gly Leu Thr Thr Gln Trp Tyr    477
AAT GAG ATC GGC CCT CTT GGG GCT ACG GGC CTC ACC ACT CAG TGG TAT   1440

Glu Tyr Ser Asp Gly Met Arg Leu Gln Asp Thr Gly Val Val Val Trp    493
GAA TAC TCG GAT GGG ATG CGG CTG CAG GAC ACG GGA GTT GTA GTG TGG   1488

Cys Lys Gly Gly Glu Ile Lys Tyr Leu Ile Thr Cys Glu Arg Glu Ala    509
TGT AAA GGT GGA GAG ATC AAA TAT CTA ATT ACA TGT GAG AGG GAA GCC   1536
```

```
Arg Tyr Leu Ala Ile Leu His Thr Arg Ala Leu Pro Thr Ser Val Val    525
AGG TAT CTG GCC ATT CTA CAC ACG AGA GCC CTG CCG ACG TCT GTA GTA   1584

Phe Glu Lys Ile Ile Asp Gly Lys Glu Gln Glu Asp Val Val Glu Met    541
TTT GAA AAA ATC ATA GAT GGA AAA GAA CAA GAG GAC GTA GTG GAA ATG   1632

Asp Asp Asn Phe Glu Leu Gly Leu Cys Pro Cys Asp Ala Lys Pro Leu    557
GAT GAT AAC TTT GAA CTC GGT CTT TGC CCG TGT GAT GCT AAA CCC TTG   1680

Val Arg Gly Lys Phe Asn Thr Thr Leu Leu Asn Gly Pro Ala Phe Gln    573
GTA AGG GGA AAA TTT AAT ACA ACA CTT CTG AAT GGG CCA GCC TTC CAG   1728

Met Val Cys Pro Ile Gly Trp Thr Gly Thr Val Ser Leu Cys His Trp    589
ATG GTT TGC CCT ATA GGA TGG ACA GGA ACT GTG AGT CTG TGT CAC TGG   1776

Ser Asn Lys Asp Thr Leu Ala Met Thr Val Val Arg Thr Tyr Lys Arg    605
TCC AAT AAG GAT ACG TTA GCC ATG ACC GTT GTA CGA ACA TAC AAG AGG   1824

His Arg Pro Phe Pro Phe Arg Gln Gly Cys Ile Thr Gln Lys Val Ile    621
CAC AGG CCT TTC CCC TTT AGG CAA GGC TGC ATT ACC CAG AAA GTC ATC   1872

Gly Gly Asp Leu Tyr Asp Cys Ala Leu Gly Gly Asn Trp Thr Cys Val    637
GGG GGA GAC CTC TAC GAC TGT GCC TTG GGA GGG AAC TGG ACT TGT GTA   1920

Pro Gly Asp Ile Leu Arg Tyr Val Asp Gly Pro Val Glu Ser Cys Lys    653
CCG GGG GAC ATA CTA CGA TAT GTA GAT GGG CCT GTC GAG TCT TGC AAG   1968

Trp Cys Gly Tyr Lys Phe His Lys Ser Glu Gly Leu Pro His Phe Pro    669
TGG TGT GGT TAC AAG TTT CAT AAA AGT GAG GGT CTG CCA CAC TTC CCA   2016

Ile Gly Lys Cys Lys Leu Lys Asn Glu Ser Gly Tyr Arg Gln Val Asp    685
ATT GGC AAG TGC AAG CTG AAG AAT GAA AGT GGC TAC AGA CAA GTA GAT   2064

Glu Thr Ser Cys Asn Arg Asp Gly Val Ala Ile Val Pro Thr Gly Ser    701
GAG ACC TCT TGC AAC AGA GAC GGT GTG GCT ATA GTA CCA ACT GGT TCG   2112

Val Lys Cys Lys Ile Gly Asp Thr Val Val Gln Val Ile Ala Met Asp    717
GTG AAA TGC AAG ATA GGG GAC ACA GTG GTG CAA GTC ATA GCA ATG GAT   2160

Asp Lys Leu Gly Pro Met Pro Cys Arg Pro Tyr Gly Met Pro ***
GAT AAG CTA GGG CCT ATG CCT TGC AGA CCA TAT GGC ATG CCA TAG GAT   2208

                                                                  2210
CC
```

SEQ ID NO : 5

TYPE DE SEQUENCE : séquence nucléotidique

LONGUEUR DE LA SEQUENCE : 3224 paires de bases

NOMBRE DE BRINS : simple

CONFIGURATION : linéaire

TYPE DE MOLECULE : ADNc pour ARN génomique

ORIGINE : BVD Osloss

RREGION TRADUITE : 9 - 3218

PROPRIETES : code pour la protéine structurale C

```
                  Met Glu Leu Ile Thr Asn Glu Leu Leu Tyr Lys Thr Tyr   13
     GA ATT CCC ATG GAG TTG ATT ACA AAT GAA CTT TTA TAC AAA ACA TAC     47
        EcoRI    NcoI

 14  Lys Gln Lys Pro Ala Gly Val Glu Glu Pro Val Tyr Asn Gln Ala Gly   29
 48  AAA CAA AAA CCC GCT GGA GTG GAG GAA CCA GTA TAT AAC CAA GCA GGT   95

 30  Asp Pro Leu Phe Gly Glu Arg Gly Val Val His Pro Gln Ala Thr Leu   45
 96  GAC CCT TTG TTT GGC GAG AGA GGA GTG GTT CAT CCG CAG GCG ACG CTA  143

 46  Lys Leu Pro His Lys Arg Gly Glu Arg Glu Val Pro Thr Asn Leu Ala   61
144  AAA CTG CCA CAT AAA AGA GGG GAG CGC GAA GTA CCT ACT AAT CTG GCG  191

 62  Ser Leu Pro Lys Arg Gly Asp Cys Arg Ser Gly Asn Ser Lys Gly Pro   77
192  TCT CTG CCA AAA AGA GGT GAC TGC AGG TCG GGT AAC AGC AAG GGA CCC  239

 78  Val Ser Gly Ile Tyr Leu Lys Pro Gly Pro Leu Phe Tyr Gln Asp Tyr   93
240  GTG AGT GGA ATC TAC CTG AAA CCG GGG CCG TTA TTC TAC CAG GAT TAC  287

 94  Lys Gly Pro Val Tyr His Arg Ala Pro Leu Glu Phe Phe Gln Glu Ala  109
288  AAA GGA CCC GTC TAT CAT AGA GCT CCA TTG GAG TTC TTT CAG GAA GCC  335

110  Ser Met Cys Glu Thr Thr Arg Arg Ile Gly Arg Val Thr Gly Ser Asp  125
336  TCT ATG TGT GAG ACA ACT AGA AGG ATT GGG AGA GTA ACT GGT AGT GAT  383

126  Gly Lys Leu Tyr His Ile Tyr Val Cys Ile Asp Gly Cys Ile Ile Val  141
384  GGT AAA TTG TAC CAC ATT TAT GTG TGC ATA GAT GGA TGC ATA ATA GTT  431

142  Lys Ser Ala Thr Lys Tyr His Gln Lys Val Leu Lys Trp Val His Asn  157
432  AAG AGC GCC ACA AAA TAT CAT CAA AAG GTA CTC AAA TGG GTC CAC AAC  479

158  Lys Leu Asn Cys Pro Leu Trp Val Ser Ser Cys Ser Asp Thr Lys Ala  173
480  AAA CTA AAT TGC CCT CTA TGG GTT TCA AGC TGC TCC GAC ACA AAA GCA  527

174  Glu Gly Ala Thr Arg Lys Lys Gln Gln Lys Pro Asp Arg Leu Glu Lys  189
528  GAA GGG GCG ACA AGA AAG AAG CAA CAA AAA CCA GAT AGG CTG GAA AAG  575

190  Gly Arg Met Lys Ile Thr Pro Lys Glu Ser Glu Lys Asp Ser Lys Thr  205
576  GGG AGG ATG AAG ATA ACT CCT AAA GAG TCG GAG AAA GAT AGT AAG ACC  623

206  Lys Pro Pro Asp Ala Thr Ile Val Val Asp Gly Val Lys Tyr Gln Val  221
624  AAA CCG CCA GAT GCT ACG ATA GTG GTA GAT GGT GTC AAA TAT CAG GTA  671

222  Lys Lys Lys Gly Lys Ile Lys Ser Lys Asn Thr Gln Asp Gly Leu Tyr  237
672  AAG AAA AAA GGG AAA ATC AAG AGT AAG AAT ACC CAG GAC GGT TTG TAC  719

238  His Asn Lys Asn Lys Pro Gln Glu Ser Arg Lys Lys Leu Glu Lys Ala  253
720  CAC AAC AAA AAT AAA CCT CAA GAG TCA CGC AAG AAA CTA GAG AAA GCC  767

254  Leu Leu Ala Trp Ala Val Ile Ala Leu Val Leu Phe Gln Val Ala Val  269
768  CTG TTG GCA TGG GCA GTA ATA GCC TTG GTT TTG TTT CAA GTC GCA GTG  815
```

```
270  Gly Glu Asn Ile Thr Gln Trp Asn Leu Gln Asp Asn Gly Thr Glu Gly   285
816  GGA GAG AAC ATA ACA CAA TGG AAC TTA CAA GAC AAT GGG ACG GAA GGA   863

286  Ile Gln Arg Ala Met Phe Gln Arg Gly Val Asn Arg Ser Leu His Gly   301
864  ATA CAA CGG GCC ATG TTC CAA AGA GGC GTA AAT AGA AGT CTG CAT GGG   911

302  Ile Trp Pro Glu Lys Ile Cys Thr Gly Val Pro Ser His Leu Ala Thr   317
912  ATC TGG CCA GAG AAA ATC TGT ACA GGT GTC CCC TCC CAC TTG GCC ACT   959

318  Asp Thr Glu Leu Lys Ala Ile His Gly Met Met Asp Ala Ser Glu Lys   333
960  GAT ACA GAA CTG AAG GCA ATT CAT GGT ATG ATG GAT GCT AGC GAG AAG  1007

334  Thr Asn Tyr Thr Cys Cys Arg Leu Gln Arg His Glu Trp Asn Lys His   349
1008 ACA AAT TAC ACA TGC TGC AGG CTC CAA CGC CAT GAG TGG AAC AAG CAT  1055

350  Gly Trp Cys Asn Trp Tyr Asn Ile Glu Pro Trp Ile Val Leu Met Asn   365
1056 GGT TGG TGC AAT TGG TAC AAT ATT GAA CCT TGG ATT GTT CTC ATG AAT  1103

366  Lys Thr Gln Ala Asn Leu Ala Glu Gly Gln Pro Pro Arg Glu Cys Ala   381
1104 AAA ACC CAA GCC AAC CTT GCT GAG GGT CAG CCA CCA AGG GAG TGT GCC  1151

382  Val Thr Cys Arg Tyr Asp Arg Asp Ser Asp Leu Asn Val Val Thr Gln   397
1152 GTT ACA TGC CGG TAT GAC CGA GAT AGT GAC CTA AAT GTA GTA ACA CAA  1199

398  Ala Arg Asn Ser Pro Thr Pro Leu Thr Gly Cys Lys Lys Gly Lys Asn   413
1200 GCT AGG AAC AGC CCC ACA CCA TTG ACA GGC TGC AAG AAA GGC AAG AAC  1247

414  Phe Ser Phe Ala Gly Val Leu Val Gln Gly Pro Cys Asn Phe Glu Ile   429
1248 TTC TCC TTT GCA GGT GTG TTG GTA CAA GGG CCT TGC AAC TTT GAA ATA  1295

430  Ala Val Ser Asp Val Leu Phe Arg Glu His Asp Cys Thr Ser Val Ile   445
1296 GCT GTA AGT GAT GTG CTG TTT AGA GAG CAC GAT TGC ACA AGT GTG ATT  1343

446  Gln Gly Thr Ala His Tyr Leu Val Asp Gly Met Thr Asn Ser Leu Glu   461
1344 CAA GGC ACG GCT CAC TAT CTG GTA GAC GGG ATG ACC AAT TCT CTA GAA  1391

462  Ser Ala Arg Gln Gly Thr Ala Lys Leu Thr Thr Trp Leu Gly Arg Gln   477
1392 AGT GCC AGG CAA GGG ACC GCA AAG TTA ACT ACT TGG TTG GGT AGG CAG  1439

478  Leu Lys Lys Leu Gly Lys Lys Leu Glu Asn Lys Ser Lys Thr Trp Phe   493
1440 CTT AAG AAA CTA GGG AAG AAA CTG GAA AAC AAG AGT AAG ACA TGG TTT  1487

494  Gly Ala Tyr Ala Ala Ser Pro Tyr Cys Glu Val Glu Arg Arg Leu Gly   509
1488 GGG GCA TAT GCA GCC TCT CCC TAC TGC GAG GTA GAA CGG AGG CTT GGT  1535

510  Tyr Ile Trp Tyr Thr Lys Asn Cys Thr Pro Ala Cys Leu Pro Lys Asn   525
1536 TAC ATC TGG TAT ACA AAG AAT TGC ACC CCT GCC TGT TTA CCA AAA AAT  1583

526  Thr Lys Ile Val Gly Pro Gly Arg Phe Asp Thr Asn Ala Glu Asp Gly   541
1584 ACA AAG ATC GTT GGC CCC GGT AGG TTC GAC ACC AAT GCG GAG GAT GGT  1631

542  Lys Ile Leu His Glu Met Gly Gly His Leu Ser Glu Val Leu Leu Leu   557
1632 AAA ATA CTG CAT GAG ATG GGG GGC CAC TTG TCA GAG GTG CTA CTA CTC  1679

558  Ser Val Val Val Leu Ser Asp Phe Ala Pro Glu Thr Ala Ser Val Val   573
1680 TCA GTG GTA GTG CTT TCC GAT TTC GCT CCA GAG ACA GCC AGT GTG GTA  1727

574  Tyr Leu Ile Leu His Phe Ser Ile Pro Gln Gly His Thr Asp Ile His   589
1728 TAT TTA ATT CTA CAT TTC TCC ATC CCA CAA GGA CAC ACT GAC ATA CAT  1775

590  Asp Cys Asp Lys Asn Gln Leu Asn Leu Thr Val Gly Leu Thr Thr Ala   605
1776 GAC TGT GAT AAA AAC CAA CTA AAC CTC ACC GTA GGA CTC ACA ACA GCT  1823

606  Glu Val Ile Pro Gly Ser Val Trp Asn Leu Gly Lys Tyr Val Cys Ile   621
1824 GAA GTA ATA CCT GGG TCA GTT TGG AAT TTG GGC AAA TAT GTT TGT ATA  1871

622  Arg Pro Asp Trp Trp Pro Tyr Glu Thr Ala Thr Phe Leu Val Phe Glu   637
1872 AGA CCA GAT TGG TGG CCT TAT GAG ACA GCC ACG TTC CTA GTG TTT GAA  1919
```

638 Glu Val Gly Gln Val Ile Arg Ile Val Leu Arg Ala Leu Arg Asp Leu 653
1920 GAG GTG GGT CAA GTG ATC AGG ATA GTC TTG AGG GCT TTA AGA GAT CTA 1967

654 Thr Arg Ile Trp Thr Ala Ala Thr Thr Thr Ala Phe Leu Val Cys Leu 669
1968 ACG CGC ATT TGG ACC GCT GCT ACG ACT ACT GCA TTC CTG GTA TGT CTG 2015

670 Val Lys Val Val Arg Gly Gln Val Leu Gln Gly Ile Leu Trp Leu Ile 685
2016 GTG AAG GTG GTG AGA GGC CAA GTG TTG CAA GGC ATA CTG TGG TTG ATA 2063

686 Leu Ile Thr Gly Ala Gln Gly Leu Pro Val Cys Lys Pro Gly Phe Tyr 701
2064 CTC ATA ACA GGG GCA CAA GGG CTC CCA GTT TGC AAA CCC GGC TTT TAC 2111

702 Tyr Ala Ile Ala Lys Asn Asn Glu Ile Gly Pro Leu Gly Ala Thr Gly 717
2112 TAC GCC ATA GCC AAA AAT AAT GAG ATC GGC CCT CTT GGG GCT ACG GGC 2159

718 Leu Thr Thr Gln Trp Tyr Glu Tyr Ser Asp Gly Met Arg Leu Gln Asp 733
2160 CTC ACC ACT CAG TGG TAT GAA TAC TCG GAT GGG ATG CGG CTG CAG GAC 2207

734 Thr Gly Val Val Val Trp Cys Lys Gly Gly Glu Ile Lys Tyr Leu Ile 749
2208 ACG GGA GTT GTA GTG TGG TGT AAA GGT GGA GAG ATC AAA TAT CTA ATT 2255

750 Thr Cys Glu Arg Glu Ala Arg Tyr Leu Ala Ile Leu His Thr Arg Ala 765
2256 ACA TGT GAG AGG GAA GCC AGG TAT CTG GCC ATT CTA CAC ACG AGA GCC 2303

766 Leu Pro Thr Ser Val Val Phe Glu Lys Ile Ile Asp Gly Lys Glu Gln 781
2304 CTG CCG ACG TCT GTA GTA TTT GAA AAA ATC ATA GAT GGA AAA GAA CAA 2351

782 Glu Asp Val Val Glu Met Asp Asp Asn Phe Glu Leu Gly Leu Cys Pro 797
2352 GAG GAC GTA GTG GAA ATG GAT GAT AAC TTT GAA CTC GGT CTT TGC CCG 2399

798 Cys Asp Ala Lys Pro Leu Val Arg Gly Lys Phe Asn Thr Thr Leu Leu 813
2400 TGT GAT GCT AAA CCC TTG GTA AGG GGA AAA TTT AAT ACA ACA CTT CTG 2447

814 Asn Gly Pro Ala Phe Gln Met Val Cys Pro Ile Gly Trp Thr Gly Thr 829
2448 AAT GGG CCA GCC TTC CAG ATG GTT TGC CCT ATA GGA TGG ACA GGA ACT 2495

830 Val Ser Leu Cys His Trp Ser Asn Lys Asp Thr Leu Ala Met Thr Val 845
2496 GTG AGT CTG TGT CAC TGG TCC AAT AAG GAT ACG TTA GCC ATG ACC GTT 2543

846 Val Arg Thr Tyr Lys Arg His Arg Pro Phe Pro Phe Arg Gln Gly Cys 861
2544 GTA CGA ACA TAC AAG AGG CAC AGG CCT TTC CCC TTT AGG CAA GGC TGC 2591

862 Ile Thr Gln Lys Val Ile Gly Gly Asp Leu Tyr Asp Cys Ala Leu Gly 877
2592 ATT ACC CAG AAA GTC ATC GGG GGA GAC CTC TAC GAC TGT GCC TTG GGA 2639

878 Gly Asn Trp Thr Cys Val Pro Gly Asp Ile Leu Arg Tyr Val Asp Gly 893
2640 GGG AAC TGG ACT TGT GTA CCG GGG GAC ATA CTA CGA TAT GTA GAT GGG 2687

894 Pro Val Glu Ser Cys Lys Trp Cys Gly Tyr Lys Phe His Lys Ser Glu 909
2688 CCT GTC GAG TCT TGC AAG TGG TGT GGT TAC AAG TTT CAT AAA AGT GAG 2735

910 Gly Leu Pro His Phe Pro Ile Gly Lys Cys Lys Leu Lys Asn Glu Ser 925
2736 GGT CTG CCA CAC TTC CCA ATT GGC AAG TGC AAG CTG AAG AAT GAA AGT 2783

926 Gly Tyr Arg Gln Val Asp Glu Thr Ser Cys Asn Arg Asp Gly Val Ala 941
2784 GGC TAC AGA CAA GTA GAT GAG ACC TCT TGC AAC AGA GAC GGT GTG GCT 2831

942 Ile Val Pro Thr Gly Ser Val Lys Cys Lys Ile Gly Asp Thr Val Val 957
2832 ATA GTA CCA ACT GGT TCG GTG AAA TGC AAG ATA GGG GAC ACA GTG GTG 2879

958 Gln Val Ile Ala Met Asp Asp Lys Leu Gly Pro Met Pro Cys Arg Pro 973
2880 CAA GTC ATA GCA ATG GAT GAT AAG CTA GGG CCT ATG CCT TGC AGA CCA 2927

974 Tyr Glu Ile Ile Pro Ser Glu Gly Pro Val Glu Lys Thr Ala Cys Thr 989
2928 TAT GAA ATC ATT CCC AGT GAG GGG CCG GTA GAA AAG ACG GCA TGT ACC 2975

990 Phe Asn Tyr Thr Lys Thr Leu Lys Asn Lys Tyr Tyr Glu Pro Arg Asp 1005
2976 TTC AAC TAC ACA AAA ACA TTA AAG AAC AAG TAT TAT GAG CCT AGG GAT 3023

```
1006  Asn Tyr Phe Gln Gln Tyr Met Leu Lys Gly Glu Tyr Gln Tyr Trp Phe  1021
3024  AAT TAT TTC CAA CAA TAC ATG TTA AAA GGG GAG TAC CAA TAT TGG TTT  3071

1022  Asp Leu Glu Ile Thr Asp His His Arg Asp Tyr Phe Ala Glu Ser Leu  1037
3072  GAC CTA GAG ATC ACT GAC CAC CAC CGG GAT TAC TTC GCT GAG TCC CTA  3119

1038  Leu Val Ile Val Val Ala Leu Leu Gly Gly Arg Tyr Val Leu Trp Leu  1053
3120  CTG GTG ATA GTG GTT GCA CTC CTG GGC GGT AGG TAC GTG CTC TGG TTA  3167

1054  Leu Val Thr Tyr Met Ile Leu Ser Glu Gln Met Thr Ser Gly Gly Pro  1069
3168  CTG GTT ACA TAT ATG ATC CTA TCA GAA CAA ATG ACC TCG GGA GGG CCC  3215

      Stop
1070  ***
3216  TGA GGA TCC
          BamHI
```

## Revendications

1. Séquence nucléotidique correspondant sensiblement à la fraction du génome du virus BVD codant pour la glycoprotéine gE2 ou séquence contenue à son intérieur et comprenant la partie codant pour ses 273 premiers acides aminés

2. Séquence nucléotidique selon la revendication 1, caractérisée en ce qu'elle correspond sensiblement aux 273 premiers codons de la fraction du génome du virus BVD codant pour la glycoprotéine gE2.

3. Séquence nucléotidique correspondant sensiblement à tout ou partie de la fraction du génome du virus BVD codant pour la glycoprotéine gE1.

4. Séquence nucléotidique contenant la séquence selon la revendication 1 ou 2 et/ou la séquence selon la revendication 3.

5. Séquence nucléotidique comprenant une fraction du génome BVD, caractérisée en ce qu'elle contient : codon d'initiation de traduction - séquence codant pour gE1 - séquence codant pour gE2 - signal de fin de copie.

6. Séquence nucléotidique comprenant une fraction du génome BVD, caractérisée en ce qu'elle contient : codon d'initiation de traduction - séquence codant pour gE1 + les 273 premiers codons de gE2 - signal de fin de copie.

7. Séquence nucléotidique comprenant une fraction du génome BVD, caractérisée en ce qu'elle contient : codon d'initialisation de traduction - séquence codant pour la fin de gE1 + les 273 premiers codons de gE2 -signal de fin de copie.

8. Séquence nucléotidique comprenant une fraction du génome BVD, caractérisée en ce qu'elle contient : codon d'initialisation de traduction - séquence codant pour les 273 premiers codons de gE2 - signal de fin de copie.

9. Séquence nucléotidique comprenant une fraction du génome BVD, caractérisée en ce qu'elle contient : codon d'initiation de traduction - Séquence codant pour gE2 - signal de fin de copie.

10. Séquence nucléotidique comprenant une fraction du génome BVD, caractérisée en ce qu'elle contient : codon d'initiation de traduction - séquence contenant le fragment codant pour les 273 premiers acides aminés de gE2 - signal de fin de copie.

11. Séquence nucléotidique comprenant une fraction du génome BVD, caractérisée en ce qu'elle contient : codon d'initiation de traduction - séquence codant pour C - séquence codant pour gE1 - séquence

codant pour gE2 - signal de fin de copie.

**12.** Fragments de la séquence nucléotidique selon l'une des revendications 1 à 4 et dont les produits de traduction sont reconnus par au moins un anticorps monoclonal neutralisant du virus BVD.

**13.** Peptide correspondant à la séquence selon l'une quelconque des revendications 1 à 12.

**14.** Peptide selon la revendication 13, caractérisé en ce qu'il est glycosylé.

**15.** Virus recombinant incorporant et exprimant la séquence selon l'une quelconque des revendications 1 à 12.

**16.** Virus selon la revendication 15, caractérisé en ce qu'il s'agit d'un Baculovirus.

**17.** Vecteur d'expression de levure incorporant et exprimant la séquence selon l'une quelconque des revendications 1 à 12.

**18.** Cellule hôte eucaryote comprenant le virus selon la revendication 15 ou 16.

**19.** Cellule hôte eucaryote selon la revendication 18, caractérisée en ce qu'elle est choisie parmi les cellules de mammifères et les cellules d'insectes.

**20.** Cellule hôte eucaryote selon la revendication 19, caractérisée en ce qu'il s'agit de Spodoptera frugiperda.

**21.** Levure comprenant le vecteur selon la revendication 17.

**22.** Levure selon la revendication 21, caractérisée en ce qu'il s'agit de Saccharomyces cerevisiae.

**23.** Vaccin contenant, dans un véhicule approprié, ou exprimant, des peptides correspondant à une séquence codant pour gE1 et gE2.

**24.** Vaccin contenant, dans un véhicule approprié, ou exprimant, des peptides correspondant à une séquence codant pour gE1 et la première moitié de gE2.

**25.** Vaccin contenant, dans un vehicule approprié, ou exprimant, des peptides correspondant à une séquence codant pour C, gE1, gE2.

**26.** Vaccin contenant, dans un véhicule approprié, ou exprimant, l'un au moins des peptides selon l'une des revendications 13 et 14.

FIG.1

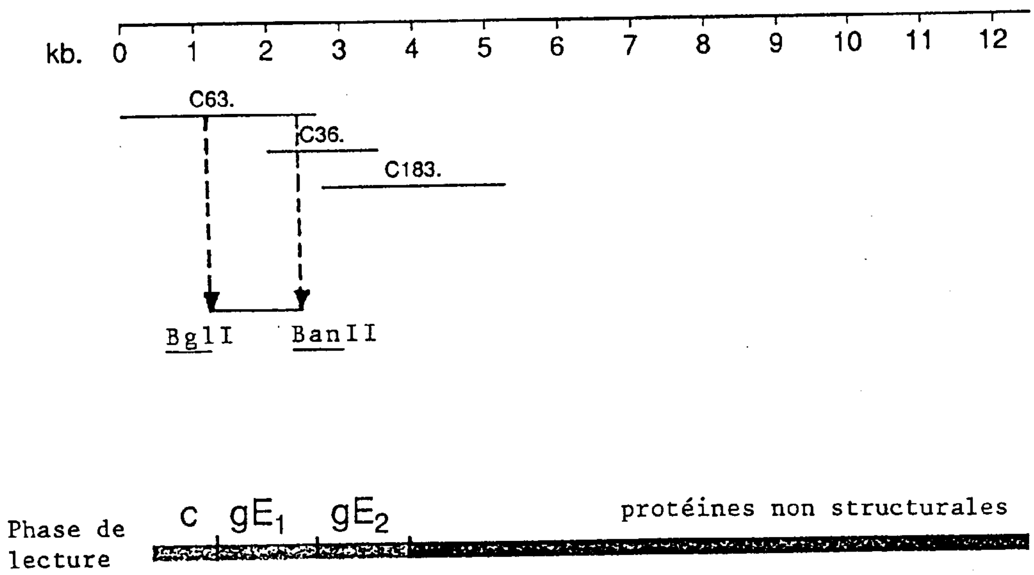
kb.
0
1
2
3
4
5
6
7
8
9
10
11
12
C63.
C36.
C183.
BglI
BanII
Phase de lecture
c
gE1
gE2
protéines non structurales

FIG. 6

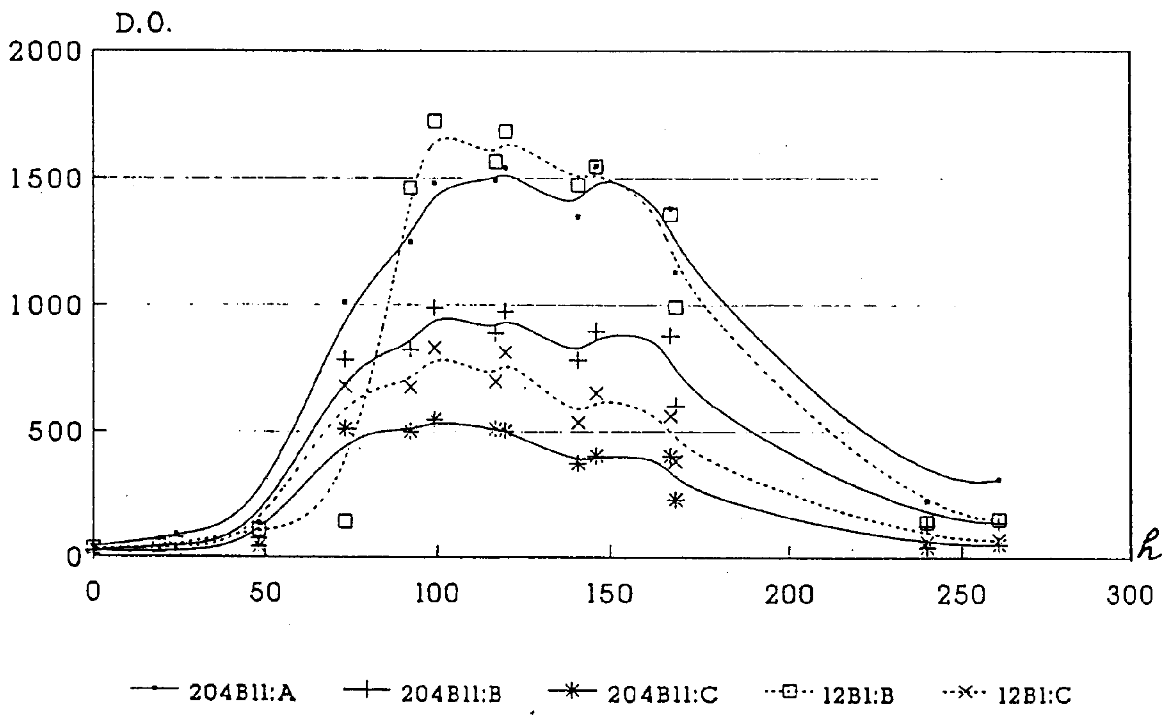
D.O.
2000
1500
1000
500
0
0
50
100
150
200
250
300
h
204B11:A
204B11:B
204B11:C
12B1:B
12B1:C

FIG. 2

A.

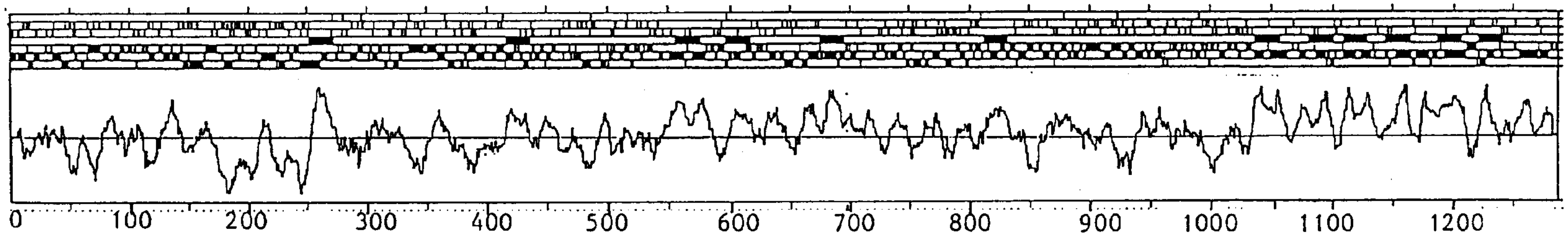


B.

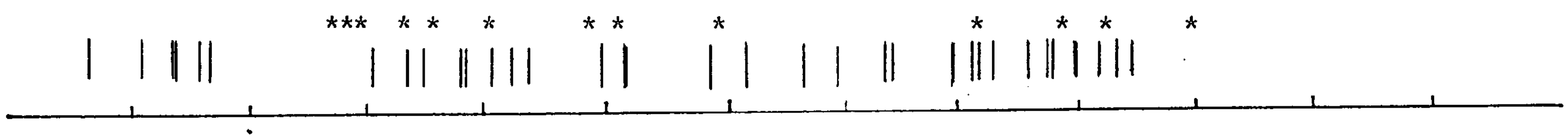

Analyse de la séquence peptidique correspondant aux protéines structurales du virus BVD Osloss.
A. Profil d'hydrophobicité.
B. *: sites potentiels de glycosylation; | : résidus cystéine.

FIG. 3

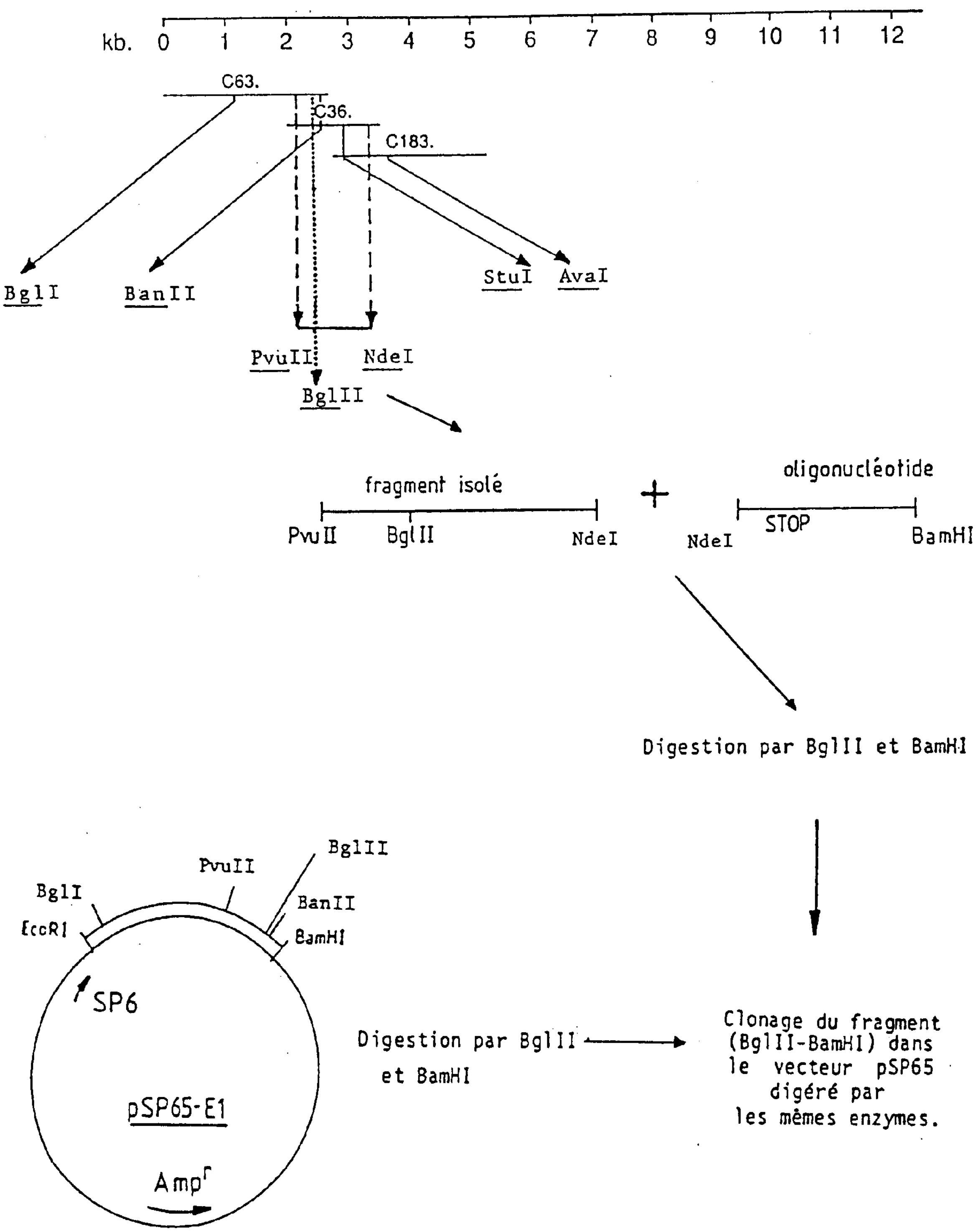
kb. 0 1 2 3 4 5 6 7 8 9 10 11 12
C63.
C36.
C183.
BglI
BanII
StuI
AvaI
PvuII
NdeI
BglII
fragment isolé
PvuII
BglII
NdeI
oligonucléotide
NdeI
STOP
BamHI
Digestion par BglII et BamHI
BglII
PvuII
BglI
BanII
EcoRI
BamHI
SP6
pSP65-E1
Amp^r
Digestion par BglII et BamHI
Clonage du fragment (BglII-BamHI) dans le vecteur pSP65 digéré par les mêmes enzymes.

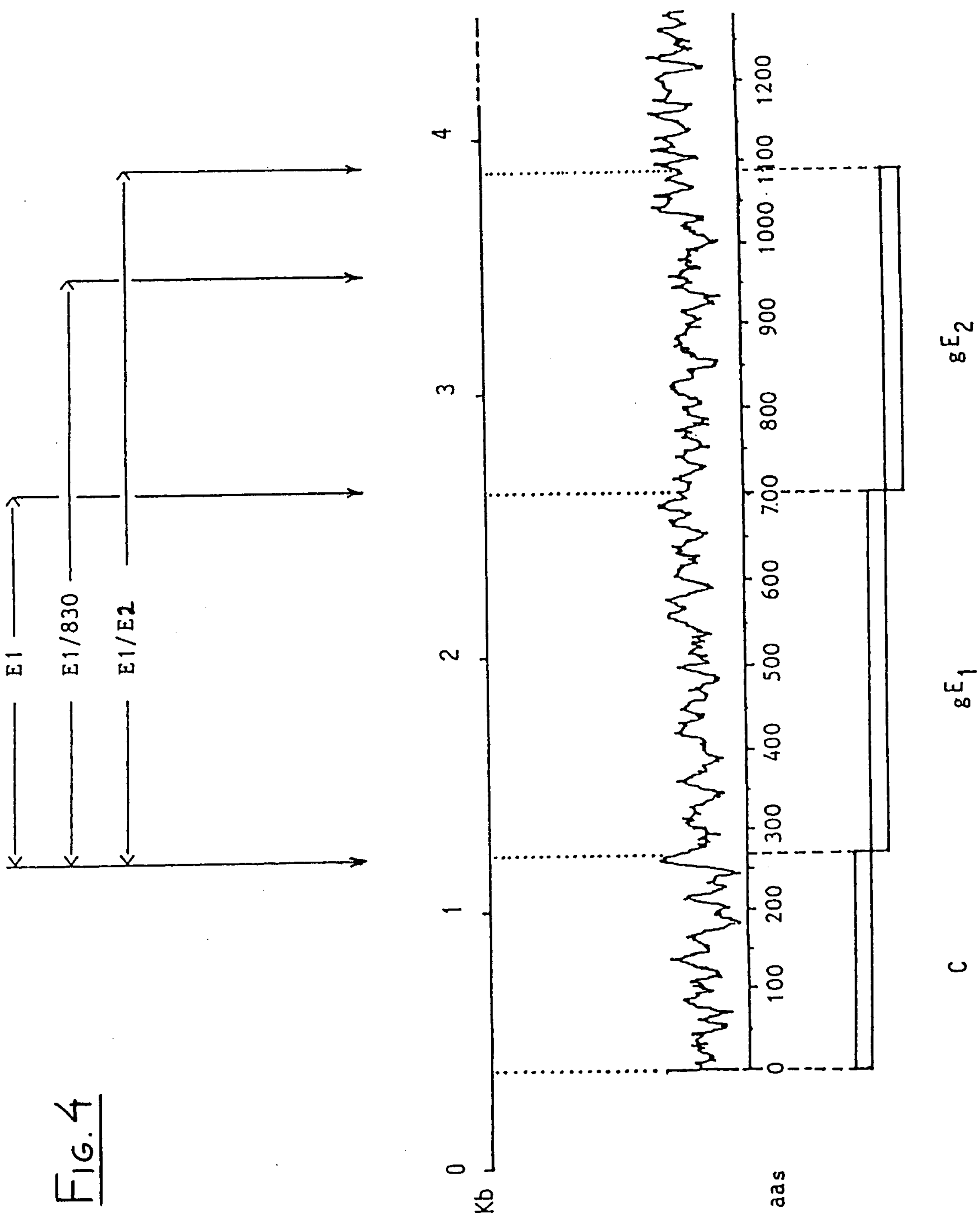
E1
E1/830
E1/E2
Kb
0
1
2
3
4
aas
0
100
200
300
400
500
600
700
800
900
1000
1100
1200
C
$gE_1$
$gE_2$

Fig. 4

FIG. 5

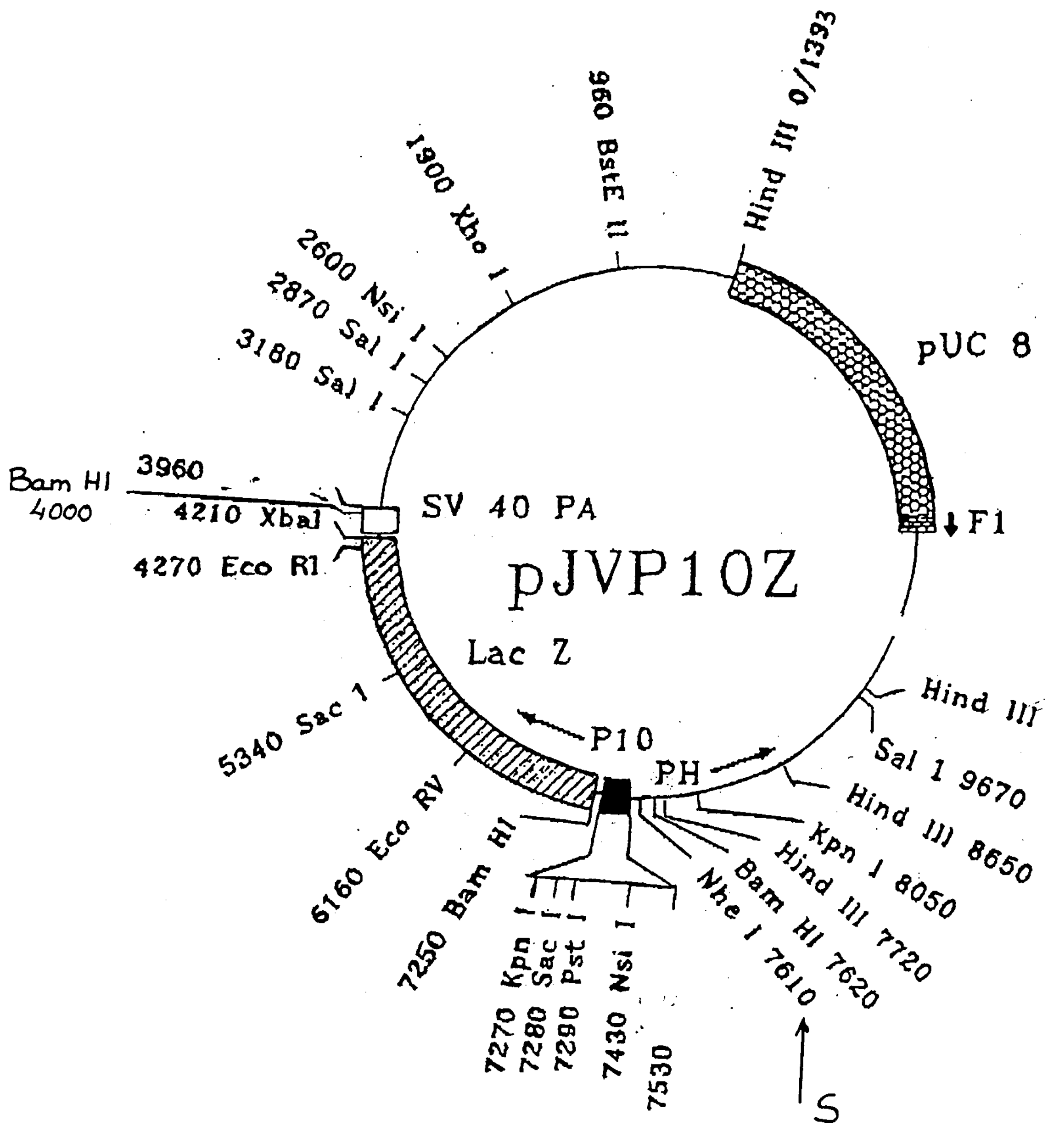

pJVP10Z
pUC 8
F1
SV 40 PA
Lac Z
P10
PH
Hind III 0/1393
960 BstE II
1900 Xho I
2600 Nsi I
2870 Sal I
3180 Sal I
3960
Bam HI 4000
4210 XbaI
4270 Eco R1
5340 Sac 1
6160 Eco RV
7250 Bam HI
7270 Kpn I
7280 Sac I
7290 Pst I
7430 Nsi I
7530
Nhe I 7610
Bam HI 7620
Hind III 7720
Kpn I 8050
Hind III 8650
Sal 1 9670
Hind III
S

Fig. 7

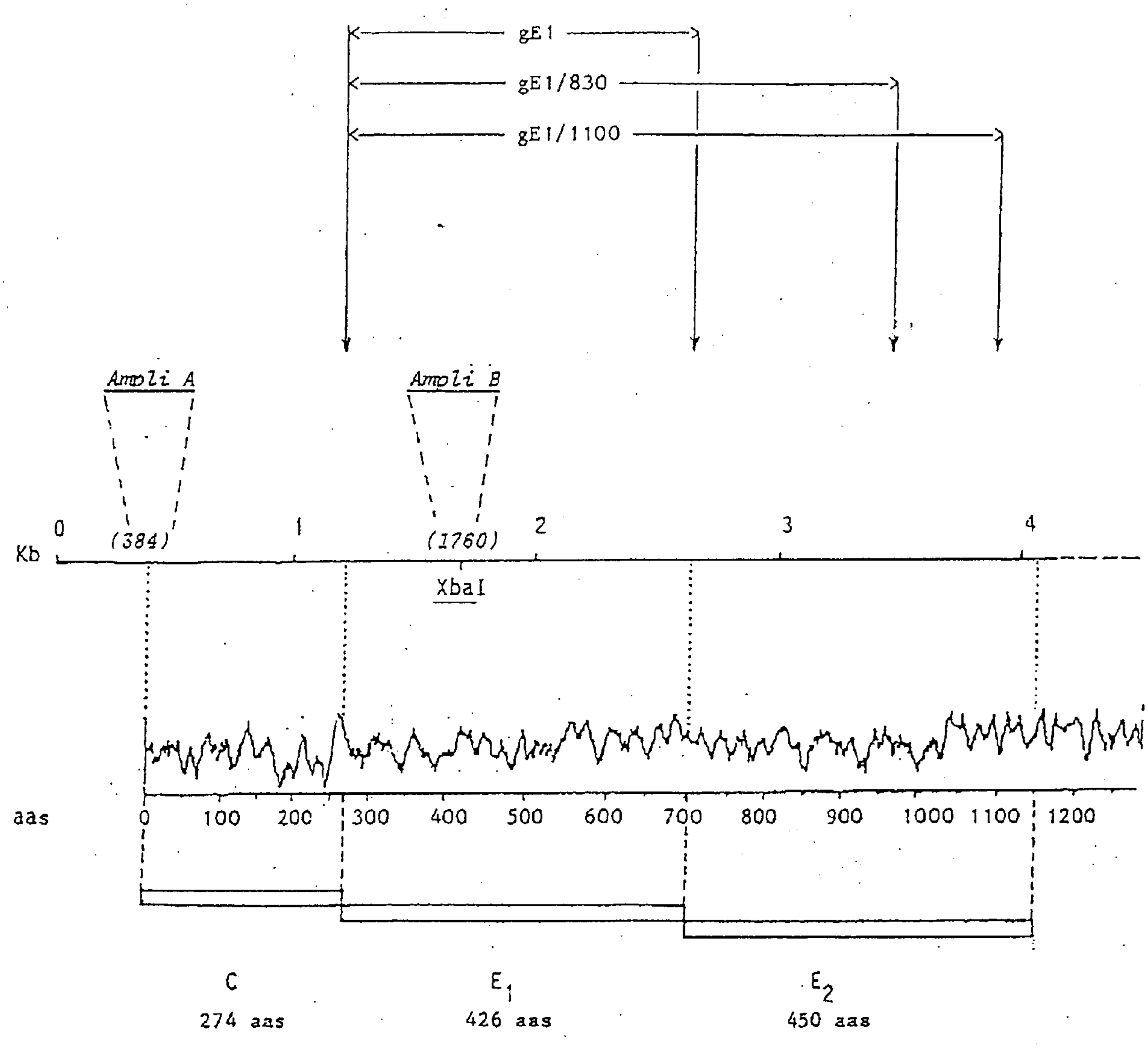
gE1
gE1/830
gE1/1100
Ampli A
Ampli B
0
1
2
3
4
Kb
(384)
(1760)
XbaI
aas
0
100
200
300
400
500
600
700
800
900
1000
1100
1200
C
274 aas
$E_1$
426 aas
$E_2$
450 aas

Office européen des brevets

# RAPPORT DE RECHERCHE EUROPEENNE

Numero de la demande

EP 92 40 1601

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | EP-A-0 236 977 (BIOTECHNOLOGY RESEARCH PARTNERS LTD) | 1,2, 8-10, 12-15, 18,19, 23,25,26 | C12N15/40<br>A61K39/12<br>C07K15/00 |
| Y | * le document en entier * | 6,7,11 | |
| X | EP-A-0 208 672 (REGION WALLONNE) | 3 | |
| Y | * page 31 - page 33 * | 6,7,11 | |
| X | VACCINES 87<br>1987, COLD SPRING HARBOUR LABORATORY<br>pages 435 - 439;<br>E.J.DUBOVI: 'expression of a glycoprotein antigen of bovine viral diarrhea virus' | 3 | |
| Y | * le document en entier * | 6,7,11 | |
| A | J. GEN. VIROL.<br>vol. 69, 1988,<br>pages 77 - 86;<br>R.O.DONIS: 'neutralizing monoclonal antibodies to bovine viral diarrhea virus' | | DOMAINES TECHNIQUES RECHERCHES (Int. Cl.5) |
| A | VIROLOGY<br>vol. 165, 1988,<br>pages 191 - 199;<br>M.C.COLLETT: 'molecular cloning and nucleotide sequence of the pestivirus bovine viral diarrhea virus' | | C12N<br>A61K<br>C07K |
| A | VIROLOGY<br>vol. 165, 1988,<br>pages 200 - 208;<br>M.S.COLLETT: 'proteins encoded by bovine viral diarrhea virus' | | |

Le présent rapport a été établi pour toutes les revendications

| Lieu de la recherche | Date d'achèvement de la recherche | Examinateur |
|---|---|---|
| LA HAYE | 17 SEPTEMBRE 1992 | SKELLY J.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)